# EUROPÄISCHE PATENTSCHRIFT

(11) **EP 2 168 442 B1**
(45) Veröffentlichungstag und Bekanntmachung des Hinweises auf die Patenterteilung: **09.11.2011**
(21) Anmeldenummer: 09170350.4
(22) Anmeldetag: 15.09.2009
(51) Int. Cl.: A23L 1/226, A23L 1/231, A23F 3/40, A23L 1/217, A23L 1/24, A23L 1/39, A23G 4/06, C07C 233/56

(54) **Geranylaminderivate der Oxalsäure**
Geranylamine derivatives of oxalic acid
Dérivés de géranylamine de l'oxyde oxalique

(30) Priorität: 26.09.2008 DE 102008042421
(43) Veröffentlichungstag der Anmeldung: 31.03.2010
(73) Patentinhaber: Symrise AG, 37603 Holzminden (DE)
(72) Erfinder: Backes, Michael, 37603 Holzminden (DE); Looft, Jan, 37603 Holzminden (DE)
(74) Vertreter: Eisenführ, Speiser & Partner

(56) Entgegenhaltungen:
- EP-A1- 1 471 052
- EP-A1- 1 878 787
- EP-A2- 1 473 287
- DE-A1- 2 462 303
- US-A1- 2004 202 619
- US-A1- 2005 010 062
- US-A1- 2006 057 268

## Beschreibung

Die vorliegende Erfindung betrifft neue Verbindungen (Geranylaminderivate der Oxalsäure) der Formel (I) oder (II), 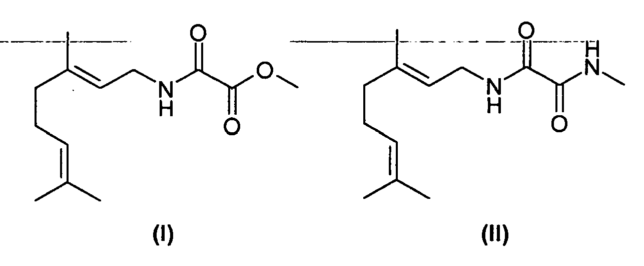 und entsprechende Mischungen, insbesondere Geschmacksstoffmischungen, die eine oder beide dieser Verbindungen sowie gegebenenfalls weitere Verbindungen umfassen oder aus diesen bestehen.

Die vorliegende Erfindung betrifft außerdem die Verwendung einer solchen Verbindung oder Mischung zum Erzeugen, Vermitteln, Modifizieren und/oder Verstärken eines Geschmackseindrucks, insbesondere einer würzigen Geschmacksnote, insbesondere eines Umami-Geschmacks.

Die Erfindung betrifft des Weiteren bestimmte Zusammensetzungen, Zubereitungen und Halbfertigwaren, welche eine geschmacklich wirksame Menge einer Verbindung der Formel (I) oder (II) oder einer entsprechenden Mischung umfassen sowie bestimmte Verfahren zum Erzeugen, Vermitteln, Modifizieren und/oder Verstärken bestimmter Geschmackseindrücke, insbesondere eines Umami-Geschmacks.

Weitere Aspekte der vorliegenden Erfindung ergeben sich aus der folgenden Beschreibung, den Ausführungsbeispielen und den Ansprüchen.

Aromastoffe, insbesondere Geschmacksstoffe, und Verbindungen mit außergewöhnlichen sensorischen Eigenschaften, die eine Amidgruppe tragen, sind seit langer Zeit bekannt. Zu den sensorisch bedeutsamen ungesättigten Amiden zählt unter anderem das Spilanthol, welches neben einer speichelanregenden und kribbelnden Wirkung einen langanhaltenden und betäubenden Effekt im Mundraum zeigt. In US 2004/0202760 und US 2004/0202619 werden in Anlehnung an die chemische Struktur des Spilanthols verschiedene Alkylidenamide vorgestellt, die ganz unterschiedliche sensorische Eindrücke wie Kribbeln, Betäuben, Bitterkeit, Mundfülle etc. bewirken. Für einige Verbindungen wie das N-Cyclopropyl-E2,Z6-nonadienamid (FEMA 4087), N-Ethyl-E2,Z6-dodecadienamid und N-Ethyl-E2,Z6-nonadienamid (FEMA 4113) wird dabei eine MSG-ähnliche Wirkung (MSG = monosodium glutamate, Natriumglutamat) bzw. ein Umamiähnlicher Geschmackseindruck angegeben. Diese Verbindungen erhielten bereits den GRAS (Generally Recognized as Safe) Status der FEMA (Flavor and Extract Manufacturers' Association) zur Verwendung in Lebensmitteln. In einer Weiterentwicklung (vgl. US 2006/057268 und US 2006/068071) wurde u. a. - angelehnt an ein Geranylgrundgerüst - N-3,7-Dimethyl-2,6-octadienyl-cyclopropylcarboxamid (FEMA 4267) als Salz- und Umami-Verstärker vorgestellt. Die Wirksamkeit von Oxalsäurederivaten, insbesondere von Geranylaminderivaten der Oxalsäure, wird in den genannten Veröffentlichungen nicht untersucht.

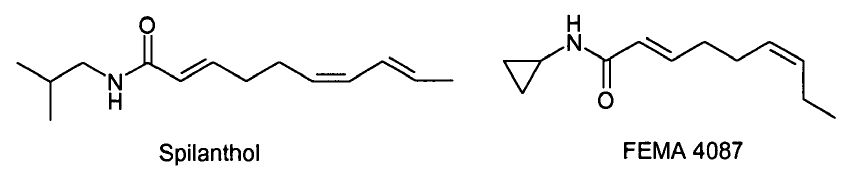

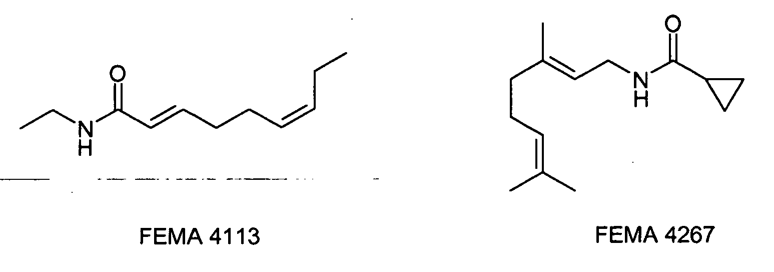

In EP 1 803 357 werden Carbamate der allgemeinen Formel 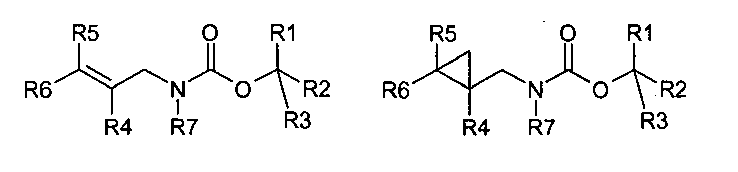 beschrieben, die geschmacksmodifizierende Eigenschaften besitzen und besonders zur Verstärkung eines Umami-Eindrucks sowie zur Verstärkung der Salzigkeit eingesetzt werden können. Es werden auch einige Beispiele vorgestellt, bei denen Geranyl bzw. Neryl der Substituent am N-Terminus ist. Allerdings werden auch hier mögliche Derivate der Oxalsäure nicht untersucht.

In US 2005/0084506 werden zahlreiche nicht-natürliche Amide zur Modifizierung des Geschmacks, insbesondere der Geschmackseigenschaften süß und würzig, beschrieben. Unter anderem werden auch diverse N,N'-Oxalamide beschrieben, denen ebenfalls der GRAS Status der FEMA zur Verwendung in Lebensmitteln erteilt worden ist.

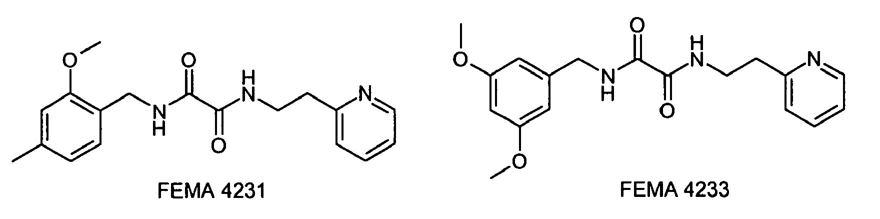

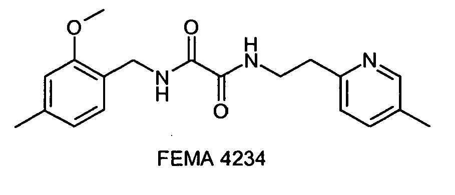

In US 2005/0084506 werden Verbindungen der allgemeinen Formel 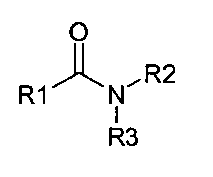 als Geschmacksstoffe beschrieben. Die erfindungsgemäßen, eingangs genannten Verbindungen der Formeln (I) und (II) sind jedoch keine Verbindungen dieser allgemeinen Formel gemäß US 2005/0084506. Das N,N'-Digeranyloxalamid **(1)** sowie das N,N'-Dineryloxalamid **(2),** welche beide unter die vorangehend genannte allgemeine Formel gemäß US 2005/0084506 fallen, zeigten in eigenen Untersuchungen keinen Umami-Charakter (vgl. hierzu Synthesebeispiele 5 und 6 der Beispiele des vorliegenden Textes).

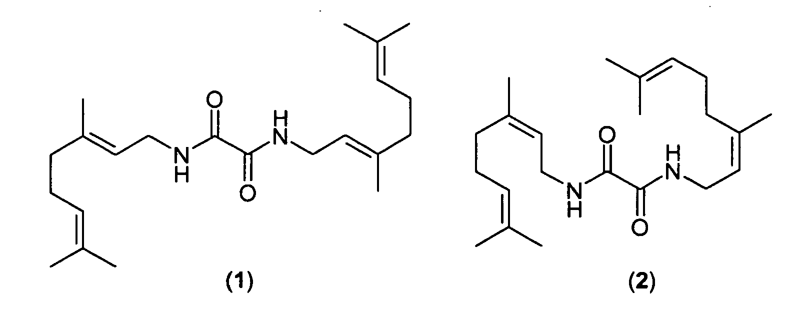

Der Ethylester (3) eines teilweise hydrierten Geranylderivats wurde bereits in der Literatur beschrieben (Maslozhirovaya Promyshlennost 1983, 4, 26-27). Im Rahmen eigener Untersuchungen wurde der entsprechende Methylester (**4**) bei der Verkostung durch ein Panel ausgebildeter Testpersonen als sehr neutral beschrieben; dem entsprechenden Methylamin (5) wurden fruchtige Noten zugeordnet (vgl. hierzu die in den Beispielen des vorliegenden Textes angeführte Tabelle 2).

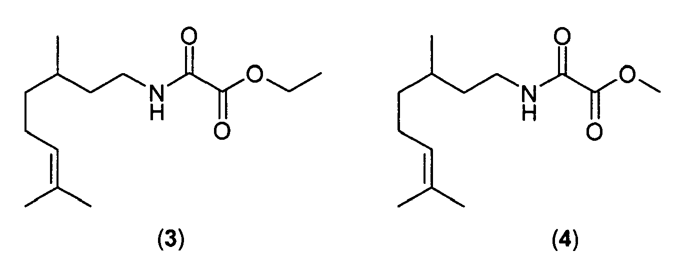

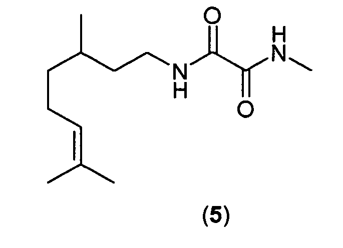

Auch die Untersuchung bzw. Verwendung des den Verbindungen der Formeln (I) und (II) entsprechenden Ethylesters bzw. Ethylamins führte in eigenen Versuchen zu keinem zufriedenstellenden Ergebnis. Während der Ethylester **(6)** durch einen muffigen, krautigen sowie chemischen Geschmack auffällt, wird das korrespondierende Ethylaminderivat **(7)** als sehr neutral bewertet.

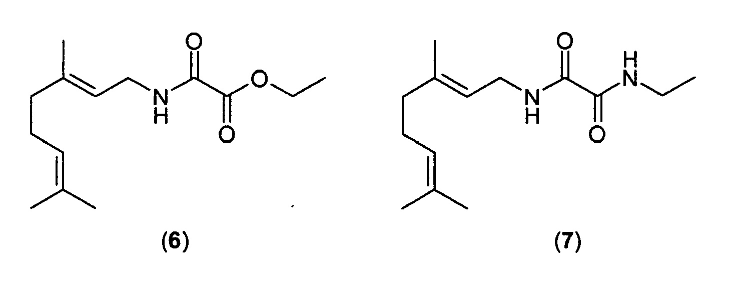

Auch bei weiteren, strukturell ähnlichen Derivaten (vgl. wiederum die unten angeführten Beispiele, insbesondere Tabelle 2) konnte kein Umami-Charakter festgestellt werden.

Es besteht prinzipiell ein ständiger Bedarf, neue Aromastoffe, insbesondere neue Geschmacksstoffe zu finden. Insbesondere besteht ein Bedarf, geschmacksaktive Verbindungen oder Verbindungen aufzufinden, die einen würzigen Geschmackseindruck erzeugen, vermitteln, modifizieren und/oder verstärken können. Ganz besonders besteht Bedarf an solchen Verbindungen, die den Geschmackseindruck "Umami" erzeugen, vermitteln, modifizieren und/oder verstärken können.

Es war daher Aufgabe der vorliegenden Erfindung, solche Verbindungen bereitzustellen.

Diese Aufgabe wird erfindungsgemäß gelöst durch die neuen Verbindungen der Formeln (I) und (II). Dementsprechend betrifft die vorliegende Erfindung die einzelnen Verbindungen der Formeln (I) und (II).

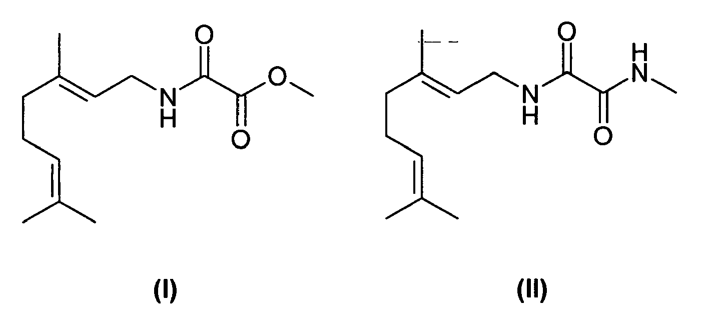

Die Verbindungen der Formeln (I) und (II) können einzeln oder aber als Mischung vorliegen. Dementsprechend betrifft die vorliegende Erfindung auch eine Mischung, insbesondere Geschmacksstoffmischung, umfassend eine Verbindung der Formel (I) und/oder eine Verbindung der Formel (II) oder bestehend aus einer Verbindung der Formel (I) und einer Verbindung der Formel (II).

Bei der Herstellung der Verbindungen (I) und (II) können in Abhängigkeit von den Reaktionsbedingungen die den Verbindungen (I) und (II) entsprechenden Nerolderivate der Formeln (III) und (IV) gegebenenfalls als Nebenprodukte entstehen und daher neben den erfindungsgemäßen Verbindungen der Formeln (I) und (II) in entsprechenden (Produkt-) Mischungen enthalten sein. Die Verbindungen der Formeln (III) und (IV) besitzen zwar selbst keinen Umami-Charakter, beeinflussen den Gesamtgeschmackseindruck einer solchen Mischung jedoch nicht negativ.

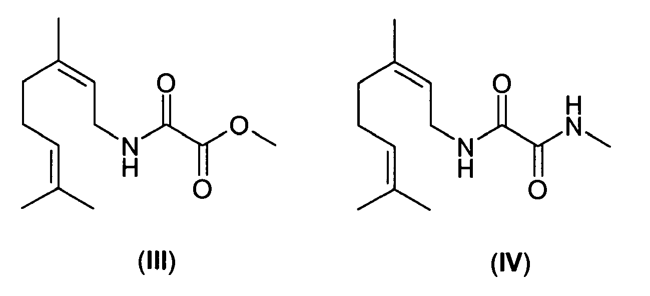

Der vorliegende Text betrifft somit zudem die neuen Verbindungen der Formeln (III) und (IV).

Eine erfindungsgemäße Mischung, insbesondere Geschmacksstoffmischung, umfasst vorzugsweise eine, mehrere oder sämtliche Verbindungen der Formeln (I), (II), (III) und (IV).

Besonders bevorzugt ist jedoch eine erfindungsgemäße Mischung, insbesondere Geschmacksstoffmischung, welche aus
- einer Verbindung der Formel (I) sowie einer Verbindung der Formel (III) oder
- einer Verbindung der Formel (II) sowie einer Verbindung der Formel (IV) oder
- sämtlichen Verbindungen der Formeln (I), (II), (III) und (IV)
besteht oder diese umfasst.

In den vorstehend genannten erfindungsgemäßen Mischungen beträgt das Gewichtsverhältnis von der Gesamtmenge an Verbindungen der Formeln (I) und (II) zur Gesamtmenge an Verbindungen der Formeln (III) und (IV) vorzugsweise 85:15 oder mehr, vorzugsweise 90:10 oder mehr, besonders bevorzugt 95:5 oder mehr.

Vorzugsweise beträgt der Gesamtanteil an Verbindungen der Formeln (I), (II), (III) und (IV) in einer erfindungsgemäßen Mischung wenigstens 0,01 ppm.

In eigenen Untersuchungen hat sich gezeigt, dass sich die erfindungsgemäßen Verbindungen der Formeln (I) und (II) bzw. die oben beschriebenen erfindungsgemäßen Mischungen besonders gut als Geschmacksstoff bzw. Geschmacksstoffmischung eignen. Besonders vorteilhaft ist dabei die Fähigkeit dieser Substanzen, einen würzigen Geschmackseindruck zu erzeugen, zu vermitteln, zu modifizieren und/oder zu verstärken. Insbesondere hat sich gezeigt, dass die erfindungsgemäßen Verbindungen bzw. Mischungen in (stark) Natriumglutamat-reduzierten, in Natriumglutamat-freien Lebensmitteln sowie in Lebensmitteln mit reduziertem Natriumchloridgehalt, so zum Beispiel in würzigen Lebensmitteln wie Tomatensuppe, Hühnersuppe, Knabbergebäck, Fertigpizza, Kartoffelchips und Popcorn, einen würzigen Geschmackseindruck, insbesondere einen Umami-Geschmackseindruck sowohl im Anfangsgeschmack (Impact) als auch in der längeranhaltenden Geschmackswahrnehmung besonders gut erzeugen, vermitteln, modifizieren und/oder verstärken können (für die Bedeutung der Begriffe "Natriumglutamat-reduziert" sowie "Natriumglutamat-frei" siehe weiter unten). Dies führt zu einem als angenehm empfunden Geschmackserlebnis, welches in vielen Fällen sogar als bevorzugt gegenüber Natriumglutamat bewertet wird.

Ein weiterer Aspekt der vorliegenden Verbindung betrifft daher die Verwendung einer erfindungsgemäßen Verbindung der Formel (I) oder (II) oder einer erfindungsgemäßen Mischung, vorzugsweise einer wie oben als bevorzugt beschriebenen Mischung, als Geschmacksstoff, vorzugsweise zum Erzeugen, Vermitteln, Modifizieren und/oder Verstärken eines Geschmackseindrucks, insbesondere eines Umami-Geschmacks. Für die bevorzugten Verbindungen bzw. Mischungen bzw. deren Gewichtsverhältnisse gilt das weiter oben Gesagte entsprechend.

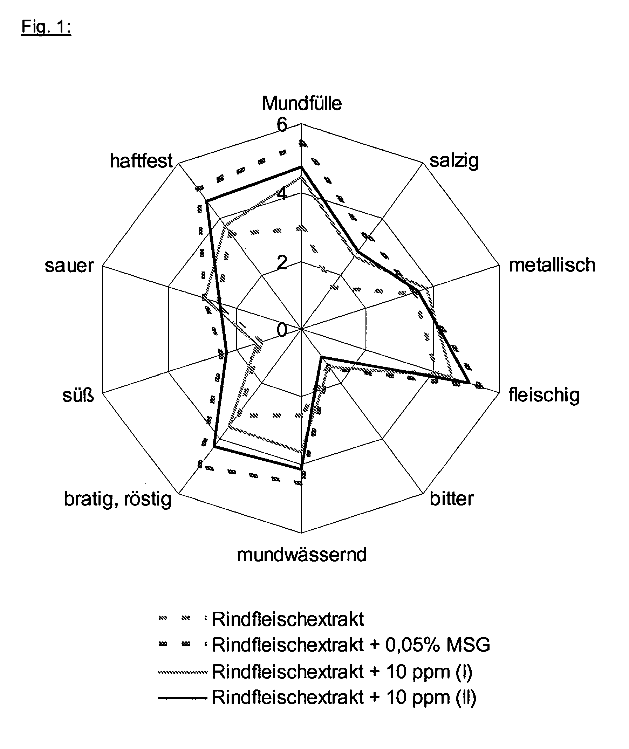

Im Vergleich zu anderen nach Umami schmeckenden Verbindungen zeichnen sich N-((*E*)*-*3,7-Dimethyl-octa-2,6-dienyl)-oxalsäureamidmethylester, d. h. eine erfindungsgemäße Verbindung der Formel (I), und *N-*((E)-3,7-Dimethyl-octa-2,6-dienyl)-*N'*-methyl-oxalamid, d. h. eine erfindungsgemäße Verbindung der Formel (II), durch einen klaren, dem Natriumglutamat (MSG, monosodium glutamate) sehr nahe kommenden Umami-Geschmack aus. Dies zeigen auch Tabelle 1 und das Spiderdiagramm in Fig. 1, in welchen ein amerikanischer Rindfleischextrakt als Base mit einer solchen Base plus Zusatz von 10 ppm einer erfindungsgemäßen Verbindung (I) bzw. 10 ppm einer erfindungsgemäßen Verbindung der Formel (II) und mit einer solchen Base plus Zusatz von 0,05 Gew.-% MSG (Natriumglutamat) verglichen wird.

**Tabelle 1**

| | Rindfleischextrakt | Rindfleischextrakt + 0,05 Gew.-% MSG | Rindfleischextrakt + 10 ppm (**I**) | Rindfleischextrakt + 10 ppm **(II)** |
|---|---|---|---|---|
| Mundfülle | 2,93 | 5,41 | 4,45 | 4,73 |
| salzig | 1,53 | 3,29 | 2,64 | 2,82 |
| metallisch | 3,40 | 3,47 | 3,82 | 3,55 |
| fleischig | 4,13 | 5,47 | 4,55 | 5,09 |
| bitter | 1,40 | 1,35 | 1,36 | 1,00 |
| mundwässernd | 2,53 | 4,53 | 3,64 | 4,14 |
| bratig, röstig | 3,13 | 5,00 | 3,55 | 4,27 |
| süß | 1,27 | 2,41 | 1,36 | 2,27 |
| sauer | 2,53 | 2,94 | 2,91 | 2,45 |
| haftfest | 3,47 | 5,06 | 3,73 | 4,64 |

Aufgrund dieses vorteilhaften sensorischen Profils betrifft die vorliegende Erfindung insbesondere die Verwendung einer erfindungsgemäßen Verbindung der Formel (I) oder (II) zum Erzeugen, Vermitteln, Modifizieren oder Verstärken eines Umami-Geschmacks. Dabei können die beiden erfindungsgemäßen Verbindungen einzeln oder in Mischung miteinander verwendet werden. Als besonders vorteilhaft zur Verwendung als Geschmacksstoff bzw. Geschmacksstoffmischung, insbesondere zum Erzeugen, Vermitteln, Modifzieren und/oder Verstärken eines Umami-Geschmacks stellte sich auch eine oben beschriebene erfindungsgemäße Mischung, also eine Mischung umfassend eine oder beide Verbindungen der Formeln (I) und (II) sowie gegebenenfalls zusätzlich (III) und/oder (IV) heraus. Dabei gilt hinsichtlich bevorzugter Verbindungen und Mischungen das oben Gesagte entsprechend.

Gemäß einem weiteren Aspekt betrifft die vorliegende Erfindung ein Verfahren zum Erzeugen, Vermitteln, Modifizieren und/oder Verstärken eines Geschmackseindrucks, insbesondere eines Umami-Geschmacks. Im erfindungsgemäßen Verfahren wird hierzu eine geschmacklich wirksame Menge einer Verbindung der Formel (I) oder (II) oder einer erfindungsgemäßen Mischung, vorzugsweise einer vorangehend als bevorzugt bezeichneten Mischung, zu weiteren Bestandteilen hinzugefügt oder mit diesen gemischt. Dadurch wird eine bestimmte Ausgangs-Substanz oder -Zusammensetzung in ihren geschmacklichen Eigenschaften entsprechend verändert. Dabei gilt hinsichtlich bevorzugter erfindungsgemäß einzusetzender Verbindungen und Mischungen das oben Gesagte entsprechend.

Wie einleitend erwähnt, betrifft die vorliegende Erfindung auch bestimmte Erzeugnisse, nämlich Zusammensetzungen, Zubereitungen und Halbfertigwaren, die eine (geschmacklich wirksame) Menge an erfindungsgemäßen Verbindungen bzw. entsprechender Mischungen umfassen. Bei solchen Erzeugnissen handelt es sich vorzugsweise um zum Verzehr geeignete Erzeugnisse. Die erfindungsgemäßen Verbindungen bzw. Mischungen eignen sich besonders gut für den Einsatz in solchen Erzeugnissen.

Im Folgenden werden einige Definitionen bzw. Beschreibungen bestimmter Erzeugniskategorien angeführt, die dem besseren Verständnis der vorliegenden Erfindung dienen sollen.

Die erfindungsgemäßen zum Verzehr geeigneten Zusammensetzungen, der Ernährung, der Mundpflege oder dem Genuss dienenden Zubereitungen oder Halbfertigwaren sind regelmäßig Produkte, die dazu bestimmt sind, in die menschliche Mundhöhle eingebracht zu werden, dort eine bestimmte Zeit zu verbleiben und anschließend entweder verzehrt (z.B. verzehrfertige Lebensmittel, siehe hierzu weiter unten) oder wieder aus der Mundhöhle entfernt zu werden (z.B. Kaugummis oder Zahnpasta). Zu diesen Produkten gehören dabei sämtliche Erzeugnisse oder Stoffe, die dazu bestimmt sind, in verarbeitetem, teilweise verarbeitetem oder unverarbeitetem Zustand vom Menschen aufgenommen zu werden. Insbesondere sind zum Verzehr geeignete Zusammensetzungen Erzeugnisse, die Lebensmitteln bei ihrer Herstellung, Verarbeitung oder Bearbeitung zugesetzt werden und dazu vorgesehen sind, in die menschliche Mundhöhle eingebracht zu werden, insbesondere mit dem besagten Lebensmittel. Demnach können solche Zusammensetzungen insbesondere auch in der Ernährung, der Mundpflege oder dem Genuss dienenden gebrauchs- oder verzehrfertigen Zubereitungen enthalten sein (der Ernährung oder dem Genuss dienende gebrauchs- oder verzehrfertige Zubereitungen sind im Rahmen des vorliegenden Textes insbesondere Lebensmittel, speziell verzehrfertige Lebensmittel (s. Definition weiter unten)). Zudem können solche Zusammensetzungen Bestandteil einer Halbfertigware sein, welche gegebenenfalls wiederum zur Herstellung von der Ernährung, der Mundpflege oder dem Genuss dienenden gebrauchs- oder verzehrfertigen Zubereitungen verwendet werden kann. Erfindungsgemäße der Ernährung, der Mundpflege oder dem Genuss dienende gebrauchs- oder verzehrfertige Zubereitungen und Halbfertigwaren werden weiter unten beschrieben.

Im Rahmen des vorliegenden Textes werden unter einem "Lebensmittei" insbesondere Stoffe verstanden, die dazu bestimmt sind, in unverändertem, zubereitetem oder verarbeitetem Zustand vom Menschen geschluckt und dann verdaut zu werden: Als Lebensmittel werden insoweit auch Umhüllungen, Überzüge oder sonstige Umschließungen verstanden, die dazu bestimmt sind, mitverschluckt zu werden, oder bei denen ein Verschlucken vorauszusehen ist. Auch bestimmte Produkte, die üblicherweise wieder aus der Mundhöhle entfernt werden (z.B. Kaugummis) sind im Rahmen des vorliegenden Textes als Lebensmittel zu verstehen, da bei ihnen nicht auszuschließen ist, dass sie zumindest teilweise geschluckt werden.

Unter einem verzehrfertigen Lebensmittel ist hierbei ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln.

Unter einer Halbfertigware ist im Zusammenhang des vorliegenden Textes ein Produkt zu verstehen, welches aufgrund eines sehr hohen Gehaltes an Aroma- und Geschmacksstoffen für die Verwendung als verzehrfertiges Lebensmittel ungeeignet ist. Erst durch Mischen mit mindestens einem weiteren Bestandteil (d.h. durch Verringern der Konzentration der betreffenden Aroma- und Geschmacksstoffe) und gegebenenfalls weitere Prozessschritte (z.B. Erwärmen, Gefrieren) wird die Halbfertigware in ein verzehrfertiges Lebensmittel überführt. Als Beispiel für Halbfertigwaren seien hier Tütensuppen, Backaromen und Puddingpulver genannt.

Unter einem Mundpflegeprodukt, d.h. einer der Mundpflege dienenden Zubereitung (Mundhygieneprodukt), wird im Rahmen des vorliegenden Textes eine dem Fachmann geläufige Formulierung zur Reinigung und Pflege der Mundhöhle und/oder des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Zubereitungen sind insbesondere Cremes, Gele, Pasten, Schäume, Emulsionen, Suspensionen, Areosole, Sprays, wie auch Kapseln, Granulate, Pastillen, Tabletten, Bonbons oder Kaugummis, ohne dass diese Aufzählung für die Zwecke dieser Erfindung limitierend verstanden werden soll.

Bevorzugte Mundpflegeprodukte (Mundhygieneprodukte) sind insbesondere solche in Form von Zahnpasta, Zahncreme, Zahngel, Zahnpulver, Zahnputzflüssigkeit, Zahnputzschaum, Mundwasser, Zahncreme und Mundwasser als 2-in-1 Produkt, Lutschbonbon, Mundspray, Zahnseide, oder Zahnpflegekaugummi. Erfindungsgemäße Ausgestaltungen der Mundpflege dienender Zubereitungen werden weiter unten beschrieben.

Kaugummis umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Stabilisatoren, Geruchskorrigentien und Aromen (z.B.: Eycalyptus-Menthol, Kirsche, Erdbeere, Grapefruit, Vanille, Banane, Citrus, Pfirsich, schwarze Johannisbeere, Tropenfrüchte, Ingwer, Kaffee, Zimt, Kombinationen (der genannten Aromen) mit Mintaromen sowie Spearmint und Pfefferminz alleine). Besonders interessant ist auch die Kombination der Aromen mit weiteren Stoffen, die kühlende, wärmende und/oder mundwässerndernde Eigenschaften aufweisen.

Im Stand der Technik sind zahlreiche unterschiedliche Kaugummibasen bekannt, wobei zwischen sogenannten "chewing gum"- und "bubble gum"-Basen zu unterscheiden ist, wobei letztere weicher sind, damit sich damit auch Kaugummiblasen bilden lassen. Gängige Kaugummibasen umfassen neben traditionell eingesetzten natürlichen Harzen oder dem Naturlatex Chicle heute zumeist Elastomere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336 US 5,601,858 oder US 6,986,709 beschrieben. Daneben umfassen Kaugummibasen weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerinmonostearat.

Im Folgenden werden erfindungsgemäße Zusammensetzungen und deren bevorzugte Ausgestaltungen beschrieben.

Eine erfindungsgemäße Zusammensetzung, insbesondere eine zum Verzehr geeignete erfindungsgemäße Zusammensetzung, besteht aus oder umfasst eine geschmacklich wirksame Menge einer erfindungsgemäßen Verbindung der Formel (I) oder (II) wie oben beschrieben oder einer erfindungsgemäßen Mischung (wie oben beschrieben) und außerdem einen oder mehrere zum Verzehr geeignete weitere Bestandteile. Dabei gilt hinsichtlich bevorzugter Verbindungen und Mischungen das oben Gesagte entsprechend. Vorzugsweise umfassen erfindungsgemäße Zusammensetzungen als weitere Bestandteile einen oder mehrere feste Trägerstoffe, vorzugsweise zum Verzehr geeignete feste Trägerstoffe.

In diesen bevorzugten erfindungsgemäßen (vorzugsweise sprühgetrockneten) Zusammensetzungen sind der eine bzw. mehrere oder sämtliche der enthaltenen Trägerstoffe ausgewählt aus der Gruppe bestehend aus Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate, vorzugsweise Maltodextrine und Dextrine), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin und Xanthan Gum. Besonders bevorzugte Trägerstoffe sind Siliciumdioxid, Gummi Arabicum und Maltodextrine, wobei hier wiederum Maltodextrine mit DE-Werten (dextrose equivalent) im Bereich von 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Mais-basierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen Zusammensetzungen, die neben der oder den erfindungsgemäßen Verbindungen der Formeln (I) und (II) bzw. wie oben beschriebenen erfindungsgemäßen Mischungen außerdem einen oder mehrere feste Trägerstoffe umfassen, können beispielsweise durch mechanische Mischvorgänge, wobei gleichzeitig auch eine Zerkleinerung vorliegender Partikel (der oben genannten Trägerstoffe) erfolgen kann, oder mittels Sprühtrocknung hergestellt werden. Wie bereits erwähnt, sind erfindungsgemäße Zusammensetzungen, die feste Trägerstoffe umfassen und mittels Sprühtrocknung hergestellt sind, besonders bevorzugt. Hinsichtlich des Verfahrens der Sprühtrocknung wird verwiesen auf US 3,159,585, US 3,971,852, US 4,532,145 und US 5,124,162.

Vorzugsweise handelt es sich bei einer erfindungsgemäßen Zusammensetzung also um eine sprühgetrocknete Zusammensetzung. Dabei sind erfindungsgemäße sprühgetrocknete Zusammensetzungen besonders bevorzugt, welche eine mittlere Partikelgröße im Bereich von 30 bis 300 µm und eine Restfeuchte von kleiner oder gleich 5 Gew.-%, bezogen auf das Gesamtgewicht der Zusammensetzung, besitzen.

Weiter bevorzugt liegt in einer erfindungsgemäßen Zusammensetzung, in der die weiteren Bestandteile feste Trägerstoffe umfassen, das Gewichtsverhältnis der Gesamtmenge an Verbindungen der Formeln (I), (II), (III) und (IV) zur Gesamtmenge an festen Trägerstoffen bezogen auf die Trockenmasse der Zusammensetzung im Bereich von 1 : 10 bis 1 : 100000, bevorzugt im Bereich von 1 : 50 bis 1 : 20000, besonders bevorzugt im Bereich von 1 : 100 bis 1 : 5000. Für die Auswahl der (vorzugsweise zum Verzehr geeigneten) festen Trägerstoffe gilt das oben Gesagte entsprechend.

Ganz besonders bevorzugt ist eine erfindungsgemäße Zusammensetzung, wobei der Gesamtanteil an Verbindungen der Formeln (I),(II), (III) und (IV) und den (zum Verzehr geeigneten) festen Trägerstoffen in der Zusammensetzung bezogen auf die Trockenmasse der Zusammensetzung 70 bis 100 Gew.-%, bevorzugt 85 bis 100 Gew.-% beträgt.

Die Erfindung betrifft auch eine (vorzugsweise sprühgetrocknete, zum Verzehr geeignete) Zusammensetzung, die neben (a) einer geschmacklich wirksamen Menge einer erfindungsgemäßen Verbindung der Formel (I) oder (II) oder einer erfindungsgemäßen Mischung sowie gegebenenfalls (b) festen Trägerstoffen zusätzlich eine Aromakomposition umfasst bzw. aus den genannten Komponenten besteht. Dabei gilt hinsichtlich bevorzugter Verbindungen, Mischungen und Trägerstoffe das oben Gesagte entsprechend.

Eine erfindungsgemäß einzusetzende Aromakomposition umfasst im Rahmen der vorliegenden Erfindung einen oder mehrere flüchtige Aromastoffe (nicht als Bestandteil der zusätzlich einzusetzenden Aromakomposition aufgefasst werden hierbei allerdings erfindungsgemäße Verbindungen der Formeln (I) und (II) sowie Verbindungen der Formeln (III) und (IV)). Der flüchtige Aromastoff ist dabei vorzugsweise eine sensorisch wirksame Komponente mit einem Dampfdruck von größer oder gleich 0,01 Pa bei 25°C, vorzugsweise einem Dampfdruck von größer oder gleich 0,025 Pa bei 25°C. Ein Großteil flüchtiger Aromastoffe weist einen Dampfdruck von größer oder gleich 1 Pa bei 25°C auf. Diese Aromastoffe werden für die Verwendung in erfindungsgemäßen Zusammensetzungen als bevorzugt angesehen.

Beispiele für Aromastoffe, die Bestandteil einer solchen Aromakomposition sein können, finden sich z.B. in H. Surburg und J. Panten, Common Fragrance and Flavor Materials, 5th. Ed., Wiley-VCH, Weinheim 2006. Es seien beispielsweise genannt: organische Säuren (gesättigt und ungesättigt) wie z.B. Buttersäure, Essigsäure, Methylbuttersäure, Capronsäure; Alkohole (gesättigt und ungesättigt) wie z.B. Ethanol, Propylenglykol, Octenol, cis-3-Hexenol, Benzylalkohol; Sulfids und Disulfide wie z.B. Dimethylsulfid, Difurfuryldisulfid, Methylthiopropanal; Thiole wie z.B. Methylfuranthiol; Pyrazine und Pyrroline wie z.B. Methylpyrazin, Acetylpyrazin, 2-Propionylpyrrolin, 2-Acetylpyrrolin.

Erfindungsgemäß kann eine solche Aromakomposition auch in Form von Reaktionsaromen (Maillard-Produkte) und/oder Extrakten bzw. ätherischen Ölen von Pflanzen oder Pflanzenteilen bzw. Fraktionen davon eingesetzt werden.

Eine weitere Ausgestaltung einer bevorzugten, erfindungsgemäßen (zum Verzehr geeigneten) Zusammensetzung ist eine Zusammensetzung, bestehend aus oder umfassend eine geschmacklich wirksame Menge einer erfindungsgemäßen Verbindungen der Formel (I) oder (II) oder einer erfindungsgemäßen Mischung (wie oben beschrieben) und außerdem als weitere (zum Verzehr geeignete) Bestandteile: Wasser, eine Ölphase, ein oder mehrere W/O-Emulgatoren, gegebenenfalls ein oder mehrere Antioxidantien und gegebenenfalls ein oder mehrere Stoffe zur Verstärkung einer antioxidativen Wirkung. Gemäß diesem Aspekt ist eine erfindungsgemäße Zusammensetzung vorzugsweise eine Wasser-in-Öl (W/O)Emulsion. Hinsichtlich bevorzugter erfindungsgemäßer Verbindungen bzw. Mischungen gilt das oben Gesagte entsprechend.

Zusammengefasst enthält eine erfindungsgemäße Zusammensetzung also als (zum Verzehr geeignete) weitere Bestandteile vorzugsweise
a) feste Trägerstoffe oder
b) feste Trägerstoffe und eine Aromakomposition oder
c) Wasser, eine Ölphase, ein oder mehrere W/O-Emulgatoren, gegebenenfalls ein oder mehrere Antioxidantien und gegebenenfalls ein oder mehrere Stoffe zur Verstärkung einer antioxidativen Wirkung.

Sofern eine erfindungsgemäße Zusammensetzung eine Zusammensetzung gemäß Alternative c) betrifft, ist es besonders bevorzugt, wenn eine solche erfindungsgemäße Zusammensetzung die folgenden Komponenten umfasst oder daraus besteht:
- 0,01 bis 0,1 Gew.-% an Verbindungen der Formeln (I), (II), (III) und (IV) (sofern jeweils enthalten),
- 5 bis 30 Gew.-%, bevorzugt 8 bis 25 Gew.-% Wasser,
- 50 bis 90 Gew.-%, bevorzugt 60 bis 80 Gew.-% einer Ölphase,
- 0,1 bis 5 Gew.-% eines verzehrbaren W/O-Emulgators jeweils bezogen auf das Gesamtgewicht der Zusammensetzung
- sowie gegebenenfalls ein oder mehrere Antioxidantien und gegebenenfalls einen oder mehrere Stoffe zur Verstärkung einer antioxidativen Wirkung.
Besonders bevorzugt besteht eine solche Zusammensetzung aus den genannten Bestandteilen in den genannten Mengen.

Die ölphase einer solchen erfindungsgemäßen W/O-Emulsion umfasst vorzugsweise ein fettes Öl und/oder eine Aromakomposition. Bevorzugt sind Ölphasen umfassend oder bestehend aus einem fetten Öl und einer Aromakomposition wie oben definiert und beschrieben.

Als fette Öle eignen sich beispielsweise Speiseöle, insbesondere Pflanzenöle. Geeignete fette Öle sind beispielsweise Borretschöl, Distelöl, Erdnussöl, Haselnussöl, Kokosöl, Kürbiskernöl, Leinöl, Maiskeimöl, Makadamianussöl, Mandelöl, Olivenöl, Palmkernöl, Pekannussöl, Pistazienkernöl, Rapsöl, Reiskeimöl, Sesamöl, Sojaöl, Sonnenblumenöl, Walnussöl oder Weizenkeimöl, oder daraus erhältliche Fraktionen. Es können auch flüssige Neutralester basierend auf mittelkettigen Fettsäuren und Glycerin verwendet werden, wie beispielsweise Miglyole (z.B. Miglyol 810, Miglyol 812). Bevorzugt sind Sonnenblumenöl, Palmkernöl und Rapsöl. Ferner bevorzugt werden fraktionierte Kokosöle verwendet, die hauptsächlich Fettsäurereste mit 6 bis 8 C-Atomen aufweisen. Diese zeichnen sich durch ihre Geschmacksneutralität sowie durch ihre gute Oxidationsstabilität aus.

Vorzugsweise wird der verzehrbare W/O-Emulgator ausgewählt aus der Gruppe bestehend aus Lecithin (E 322), Mono- und Diglyceride von Speisefettsäuren (E 471), Essigsäuremonoglyceride (E 472a), Milchsäuremonoglyceride (E 472b), Citronensäuremonoglyceride (E 472c), Weinsäuremonoglyceride (E 472d), Diacetylweinsäuremonoglyceride (E 472e) und Sorbitanmonostearat (E 491).

Geeignete Antioxidantien und Stoffe, welche die antioxidative Wirkung verstärken können, sind die natürlich vorkommenden Tocopherole und deren Derivate, Tocotrienole, Flavonoide, Ascorbinsäure und ihre Salze, alpha-Hydroxysäuren (z.B. Citronensäure, Milchsäure, Äpfelsäure, Weinsäure) und deren Na-, K- und Ca-Salze, aus Pflanzen isolierte Inhaltsstoffe, Extrakte bzw. Fraktionen davon z.B. aus Tee, Grüntee, Algen, Traubenkernen, Weizenkeimen, Rosmarin, Oregano, Flavonoide, Quercetin, phenolische Benzylamine. Weiterhin sind als Antioxidantien Propylgallat, Octylgallat, Dodecylgallat, Butylhydroxyanisol (BHA), Butylhydroxytoluol (BHT), Lecithine, Mono- und Diglyceride von Speisefettsäuren verestert mit Citronensäure, Orthophosphate und Na-, K- und Ca-Salze der Monophosphorsäure und Ascorbylpalmitat geeignet.

Die erfindungsgemäßen W/O-Emulsionen eignen sich vorteilhafterweise besonders zum Aufbringen auf Lebensmitteloberflächen, wobei die Lebensmittel vorzugsweise einen Wassergehalt von 10 Gew.-% oder weniger, besonders bevorzugt von 5 Gew.-% oder weniger aufweisen. In einer bevorzugten Ausführungsform weist die erfindungsgemäße W/O-Emulsion hierfür bei Aufbringungstemperatur eine ausreichend niedrige Viskosität auf, so dass ein Aufbringen der W/O-Emulsion mittels Sprühen möglich ist. Bevorzugte Lebensmittel, auf deren Oberflächen eine erfindungsgemäße W/O-Emulsion aufgebracht werden kann sind beispielsweise Cracker, Chips (z.B. auf Basis von Kartoffeln, Mais, Getreide oder Brot), extrudierte Knabberartikel (Snacks, z.B. Flips) oder Laugengebäck (z.B. Salzstangen). Erfindungsgemäße W/O-Emulsionen werden regelmäßig in einer Menge von 0,5 bis 6 Gew.-% auf die Lebensmitteloberflächen aufgebracht, bezogen auf das Gesamtgewicht des Lebensmittels.

Wie bereits erwähnt, betrifft die vorliegende Erfindung neben den vorangehend beschriebenen erfindungsgemäßen Zusammensetzungen auch insbesondere der Ernährung, der Mundpflege oder dem Genuss dienende (i) gebrauchs- oder verzehrfertige Zubereitungen und (ii) Halbfertigwaren.

Demnach betrifft ein weiterer Aspekt der vorliegenden Erfindung eine der Ernährung, der Mundpflege oder dem Genuss dienende (i) gebrauchs- oder verzehrfertige Zubereitung oder (ii) Halbfertigware, umfassend
- eine geschmacklich wirksame Menge einer erfindungsgemäßen Verbindung der Formel (I) oder (II) oder einer erfindungsgemäßen Mischung (jeweils wie oben beschrieben) oder
- eine erfindungsgemäße Zusammensetzung (wie oben beschrieben).

Vorzugsweise werden hierfür eine erfindungsgemäße Zusammensetzung, vorzugsweise eine vorstehend als bevorzugt bezeichnete Zusammensetzung, oder eine geschmacklich wirksame Menge einer erfindungsgemäßen Verbindung der Formel (I) oder (II) bzw. einer erfindungsgemäßen Mischung (wie oben beschrieben) in der Ernährung, der Mundpflege oder dem Genuss dienenden (i) gebrauchs- oder verzehrfertigen Zubereitungen oder (ii) Halbfertigwaren eingesetzt, besonders in der Ernährung, der Mundpflege oder dem Genuss dienenden Natriumglutamat-reduzierten oder -freien Zubereitungen oder entsprechenden Halbfertigwaren, insbesondere in der Ernährung oder dem Genuss dienenden Natriumglutamat-reduzierten oder -freien Zubereitungen oder entsprechenden Halbfertigwaren. Dabei gilt hinsichtlich bevorzugter erfindungsgemäßer Verbindungen und Mischungen das oben Gesagte entsprechend.

Eine erfindungsgemäße der Ernährung, der Mundpflege oder dem Genuss dienende (gebrauchs- oder verzehrfertige) Zubereitung (oder Halbfertigware) ist also vorzugsweise eine Natrium-reduzierte oder Natrium-freie Zubereitung (oder Halbfertigware). Der Begriff "Natriumglutamat-reduziert" bedeutet im Rahmen der vorliegenden Erfindung, dass die erfindungsgemäße Zubereitung oder Halbfertigware deutlich weniger Natriumglutamat enthält, als in der üblichen Zubereitung oder Halbfertigware (d. h. in einer nicht erfindungsgemäßen Zubereitung oder Halbfertigware ohne erfindungsgemäße Verbindungen der Formeln (I) und (II) bzw. entsprechende erfindungsgemäße Mischungen) enthalten ist. Der Natriumglutamatgehalt einer solchen bevorzugten erfindungsgemäßen Zubereitung oder Halbfertigware liegt demnach vorzugsweise unter dem Natriumglutamatgehalt der üblichen Zubereitung bzw. Halbfertigware, vorzugsweise um 5 bis weniger als 100 Gew.-%, bevorzugt um 10 bis 50 Gew.-%, besonders bevorzugt um 20 bis 50 Gew.-% unter dem Natriumglutamatgehalt der üblichen Zubereitung bzw. Halbfertigware.

Sofern neben einer Verbindung der Formel (I) oder (II) bzw. einer entsprechenden erfindungsgemäßen Mischung auch Natriumglutamat in einer erfindungsgemäßen Zubereitung oder Halbfertigware vorliegt, liegt das Gewichtsverhältnis der Gesamtmenge an Verbindungen der Formeln (I), (II), (III) und (IV) zur Gesamtmenge an Natriumglutamat vorzugsweise im Bereich von 1 : 1 bis 1 : 200. Die Gesamtmenge der Verbindungen der Formeln (I), (II), (III) und (IV) ist demnach in solchen Fällen vorzugsweise gleich oder geringer als die von Natriumglutamat (MSG), da insbesondere die erfindungsgemäßen Verbindungen der Formeln (I) und (II) sensorisch vorteilhafterweise um den Faktor 10 bis 100 aktiver sind als Natriumglutamat.

Demnach umfasst eine weitere bevorzugte, erfindungsgemäße der Ernährung, der Mundpflege oder dem Genuss dienende (i) gebrauchs- oder verzehrfertige Zubereitung oder (ii) Halbfertigware (wie oben beschrieben) Natriumglutamat, wobei das Gewichtsverhältnis der Gesamtmenge an Verbindungen der Formeln (I), (II), (III) und (IV) zur Gesamtmenge an Natriumglutamat vorzugsweise im Bereich von 1 : 1 bis 1 : 200 liegt.

Insbesondere bevorzugt ist also eine erfindungsgemäße der Ernährung, der Mundpflege oder dem Genuss dienende (i) gebrauchs- oder verzehrfertige Zubereitung oder (ii) Halbfertigware, umfassend
(a) kein Natriumglutamat
   oder
(b) Natriumglutamat, wobei das Gewichtsverhältnis der Gesamtmenge an Natriumglutamat zur Gesamtmenge an Verbindungen der Formeln (I), (II), (III) und (IV) (jeweils sofern enthalten) vorzugsweise im Bereich von 1 : 1 bis 1 : 200 liegt.

Sofern die vorliegende Erfindung der Ernährung, der Mundpflege oder dem Genuss dienende (i) gebrauchs- oder verzehrfertige Zubereitungen betrifft, umfassen diese vorzugsweise eine Gesamtmenge von 0,01 ppm bis 100 ppm, bevorzugt 0,1 ppm bis 50 ppm, besonders bevorzugt 0,5 ppm bis 30 ppm an Verbindungen der Formeln (I), (II), (III) und (IV) bezogen auf das Gesamtgewicht der gebrauchs- oder verzehrfertigen Zubereitung.

Sofern die vorliegende Erfindung erfindungsgemäße (ii) Halbfertigwaren betrifft, umfassen diese vorzugsweise 10 ppm bis 100000 ppm, bevorzugt 25 ppm bis 5000 ppm, besonders bevorzugt 50 ppm bis 1200 ppm an Verbindungen der Formeln (I), (II), (III) und (IV), bezogen auf das Gesamtgewicht der (ii) Halbfertigware. Erfindungsgemäße (ii) Halbfertigwaren, insbesondere vorstehend als bevorzugt beschriebene Halbfertigwaren, eignen sich insbesondere zur Herstellung von (vorzugsweise erfindungsgemäßen) der Ernährung oder dem Genuss dienenden (i) gebrauchs- oder verzehrfertigen Zubereitungen.

Besonders bevorzugt ist auch eine Natriumglutamat-reduzierte Zubereitung, insbesondere eine erfindungsgemäße der Ernährung, der Mundpflege oder dem Genuss dienende (i) gebrauchs- oder verzehrfertige Zubereitung (wie oben beschrieben), umfassend Natriumglutamat,
wobei
die Menge an Natriumglutamat nicht ausreicht, um in einer Vergleichszubereitung, die keine Verbindung der Formeln (I) oder (II) umfasst, aber ansonsten identisch zusammengesetzt ist, als (befriedigender) Umami-Geschmack wahrgenommen zu werden (wie weiter unten beschrieben wird ein Umami-Geschmack auch schon bei niedriger MSG-Konzentration wahrgenommen), und
die Menge an Verbindung(en) der Formel (I) und/oder (II) bzw, einer erfindungsgemäßen Mischung (wie oben beschrieben) bzw. einer erfindungsgemäßen Zusammensetzung (wie oben beschrieben) ausreicht, um einen (befriedigenden) Umami-Geschmackseindruck zu erreichen. Das heißt, die Gesamtmenge an (a) Verbindung(en) der Formel (I) und/oder (II) bzw. einer erfindungsgemäßen Mischung bzw. einer erfindungsgemäßen Zusammensetzung und (b) Natriumglutamat reicht aus, um insgesamt einen (befriedigenden) Umami-Geschmackseindruck zu erreichen.

Insbesondere bevorzugt ist eine Natriumglutamat-reduzierte Zubereitung, insbesondere eine erfindungsgemäße der Ernährung, der Mundpflege oder dem Genuss dienende (i) gebrauchs- oder verzehrfertige Zubereitung (wie oben beschrieben), umfassend Natriumglutamat, wobei die Menge an erfindungsgemäßen Verbindung(en) der Formel (I) und/oder (II) bzw. einer erfindungsgemäßen Mischung (wie oben beschrieben) bzw. einer erfindungsgemäßen Zusammensetzung (wie oben beschrieben) in der Zubereitung ausreicht, um einen identischen oder einen verstärkten Geschmackseindruck, insbesondere Umami-Geschmack zu erzeugen oder zu vermitteln wie eine Zubereitung, welche keine erfindungsgemäßen Verbindung(en) der Formel (I) und/oder (II) enthält, jedoch wenigstens die 1,05-fache Menge an Natriumglutamat, ansonsten aber identisch zusammengesetzt ist.

Ebenfalls bevorzugt ist eine Natriumglutamat-freie erfindungsgemäße der Ernährung, der Mundpflege oder dem Genuss dienende (i) gebrauchs- oder verzehrfertige Zubereitung.

Insbesondere können erfindungsgemäße (ii) Halbfertigwaren zur additiven Verstärkung des Umami-Geschmacks von Natriumglutamat-reduzierten Nahrungs- und Genussmitteln und auch direkt als Würzmittel für die industrielle oder nicht-industrielle Zubereitung von Nahrungs- und/oder Genussmitteln dienen.

Eine erfindungsgemäße (ii) Halbfertigware (wie oben beschrieben) umfasst neben den Verbindungen der Formeln (I), (II), (III) und (IV) (vorzugsweise enthalten in einer Gesamtmenge von 10 ppm bis 100000 ppm, bevorzugt 25 ppm bis 5000 ppm, besonders bevorzugt 50 ppm bis 1200 ppm), vorzugsweise
- 0 bis 10 Gew.-% (vorzugsweise 0,00001 Gew.-% bis 10 Gew.-% oder, besonders bevorzugt, kein Natriumglutamat), bevorzugt 0,0001 Gew.-% bis 5 Gew.-%, besonders bevorzugt 0,001 Gew.-% bis 2 Gew.-% an Natriumglutamat,
   sowie
- 0 bis 90 Gew.-% (vorzugsweise 0,00001 Gew.-% bis 90 Gew.-% oder keine Aromakomposition), bevorzugt 0,0001 Gew.-% bis 90 Gew.-%, bevorzugt 0,001 bis 30 Gew.-% einer Aromakomposition (wie oben definiert und beschrieben; bei dieser quantitativen Betrachtung wird jeder in der Halbfertigware enthaltene Aromastoff, mit Ausnahme der Verbindungen der Formeln (I), (II), (III) und (IV) und Natriumglutamat, der Aromakomposition zugerechnet),
   jeweils bezogen auf das Gesamtgewicht der Halbfertigware.

Wie bereits erwähnt, eignen sich erfindungsgemäße (ii) Halbfertigwaren, insbesondere vorstehend als bevorzugt bezeichnete Halbfertigwaren, besonders gut zur Herstellung von erfindungsgemäßen der Ernährung oder dem Genuss dienenden (i) gebrauchs- oder verzehrfertigen Zubereitungen.

Der Ernährung oder dem Genuss dienende (i) (gebrauchs- oder verzehrfertige) Zubereitungen oder (ii) Halbfertigwaren im Sinne der vorliegenden Erfindung sind insbesondere (vorzugsweise jeweils mit reduziertem Gehalt an Natriumglutamat) Backwaren (z.B. Brot, Trockenkekse, Kuchen, sonstiges Gebäck), Getränke (z.B. Gemüsesäfte, Gemüsesaftzubereitungen), Instantgetränke (z.B. Instant-Gemüsegetränke), Fleischprodukte (z.B. Schinken, Frischwurst- oder Rohwurstzubereitungen, gewürzte oder marinierte Frischoder Pökelfleischprodukte), gewürzte oder marinierte Fischprodukte (z.B. Surimi), Eier oder Eiprodukte (Trockenei, Eiweiß, Eigelb), Getreideprodukte (z.B. vorgegarte Fertigreis-Produkte, Reismehlprodukte, Hirse- und Sorghum-Produkte, rohe oder vorgegarte Nudeln und Pastaprodukte), Milchprodukte (z.B. Frischkäse, Weichkäse, Hartkäse, Milchgetränke, Molke, Butter, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fischsoßen wie z.B. Anchovis-Soßen, Austernsoßen, Gemüsezubereitungen (z.B. Ketchup, Soßen, Trockengemüse, Tiefkühlgemüse, vorgegarte Gemüse, in Essig eingelegte Gemüse, Gemüsekonzentrate oder -pasten, eingekochte Gemüse, Kartoffelzubereitungen), Knabberartikel (z.B. gebackene oder frittierte Kartoffelchips oder Kartoffelteigprodukte, Brotteigprodukte, Extrudate auf Mais-, Reis- oder Erdnussbasis), Produkte auf Fett- und Ölbasis oder Emulsionen derselben (z.B. Mayonnaise, Aufstrich, Remoulade, Dressings, Würzzubereitungen), sonstige Fertiggerichte und Suppen (z.B. Trockensuppen, Instant-Suppen, vorgegarte Suppen), Brühwürfel, Soßen (Instant-Soßen, Trockensoßen, Fertigsoßen), Gewürze oder Gewürzzubereitungen (z.B. Senfzubereitungen, Meerrettichzubereitungen), Würze, Würzmittel, Würzmischungen sowie insbesondere Aufstreuwürzungen (englisch: Seasonings), die beispielsweise im Snackbereich Anwendung finden.

Die erfindungsgemäßen Zubereitungen im Rahmen der vorliegenden Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die erfindungsgemäßen Zusammensetzungen, Zubereitungen bzw. Halbfertigwaren (jeweils wie oben beschrieben) werden vorzugsweise hergestellt, indem die erfindungsgemäßen Verbindungen der Formeln (I) oder (II) bzw. entsprechende erfindungsgemäße Mischungen (wie oben beschrieben) in Mischungen aus Ethanol und gegebenenfalls demineralisiertem und/oder gereinigtem Wasser gelöst und gemischt werden. Anschließend werden die Lösungen vorzugsweise durch einen Trocknungsprozess, vorzugsweise einen Sprühtrocknungs-, Vakuumgefriertrockungs-, Umkehrosmose-, Eindampf- oder anderen Konzentrationsprozess oder eine Kombination der genannten Prozesse, in eine (zumindest nahezu) feste Zubereitung überführt. Dabei kann die Trocknung unter Zuhilfenahme von Trägerstoffen (z.B. Stärke, Stärkederivate, Maltodextrin, Kieselgel, siehe oben) oder Hilfsstoffen (z.B. Pflanzengummen, Stabilisierungsmittel) erfolgen. Vorzugsweise erfolgt die Trocknung mittels Sprühtrocknung oder Vakuumgefriertrocknung.

Bevorzugte erfindungsgemäße Zusammensetzungen, Zubereitungen und Halbfertigwaren sind (je nach Anwendung) Produkte ausgewählt aus der Gruppe bestehend aus Würze, Würzmischungen, Würzmittel, Brühwürfel, Instant-Suppen, Instant-Soßen, vegetarische Fertiggerichte, fleischhaltige Fertiggerichte, Fischsoßen wie z.B. Anchovis-Soßen, Austernsoßen und Sojasoßen.

Gemäß einer weiteren bevorzugten Ausführungsform werden zur Herstellung erfindungsgemäßer Zusammensetzungen, Zubereitungen und Halbfertigwaren erfindungsgemäße Verbindungen der Formeln (I) oder (II) bzw. erfindungsgemäße Mischungen (wie oben beschrieben) sowie gegebenenfalls andere Bestandteile zunächst in Emulsionen, in Liposomen (z.B. ausgehend von Phosphatidylcholin) in Microsphären, in Nanosphären oder auch in Kapseln, Granulate oder Extrudate aus einer für Lebens- und Genussmittel geeigneten Matrix (z.B. aus Stärke, Stärkederivaten, Cellulose oder Cellulosederivaten wie Hydroxypropylcellulose, anderen Polysacchariden wie Alginat, natürlichen Fetten, natürlichen Wachsen wie Bienenwachs oder Carnaubawachs oder aus Proteinen wie Gelatine) eingearbeitet.

Besonders bevorzugt sind im Rahmen der vorliegenden Erfindung erfindungsgemäße Zusammensetzungen, Zubereitungen und Halbfertigwaren, bei denen die Matrix so gewählt ist, dass die erfindungsgemäßen Verbindungen der Formeln (I) oder (II) bzw. erfindungsgemäße Mischungen (wie jeweils oben beschrieben) verzögert von der Matrix freigegeben werden, so dass man eine langanhaltende Wirkung erhält. Als Matrix können hier z.B. natürliche Fette, natürliche Wachse (z.B. Bienenwachs, Carnaubawachs) oder auch natürliche Ballaststoffe (Weizenfasern, Apfelfasern, Haferfasern, Orangenfasern) verwendet werden.

In einem weiteren bevorzugten Herstellungsverfahren werden erfindungsgemäße Verbindungen der Formeln (I) oder (II) bzw. erfindungsgemäße Mischungen (wie oben beschrieben) mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt. Für die erfindungsgemäßen Verbindungen bzw. Mischungen gilt hierbei jeweils das oben Gesagte entsprechend. Erfindungsgemäße der Ernährung oder dem Genuss dienende (gebrauchs- oder) verzehrfertige Zubereitungen bzw. erfindungsgemäße Halbfertigwaren können als weitere Bestandteile übliche Grund-, Hilfs- und Zusatzstoffe für Nahrungs- oder Genussmittel umfassen, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbinsäure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße Zusammensetzungen, Zubereitungen oder Halbfertigwaren eine Aromakomposition, um den Geschmack und/oder den Geruch abzurunden und zu verfeinern. Eine erfindungsgemäße Zusammensetzung, die als weitere Bestandteile einen oder mehrere feste Trägerstoffe und eine Aromakomposition umfasst, wurde bereits weiter oben beschrieben.

Vorzugsweise umfassen auch erfindungsgemäße Zubereitungen oder Halbfertigwaren (wie oben beschrieben) eine Aromakomposition, um den Geschmack und/oder den Geruch abzurunden und zu verfeinern. Wie bereits weiter oben erwähnt, gilt, dass hierbei erfindungsgemäße Verbindungen der Formeln (I) und (II) sowie Verbindungen der Formeln (III) und (IV) nicht als Bestandteil der zusätzlich einzusetzenden Aromakomposition aufgefasst werden. Geeignete Aromakompositionen enthalten prinzipiell z.B. synthetische, natürliche oder naturidentische Aroma-, Riech- und Geschmacksstoffe, Reaktionsaromen, Raucharomen oder andere aromagebende Zubereitungen (z.B. Protein[teil]hydrolysate, Grillaromen, Pflanzenextrakte, Gewürze, Gewürzzubereitungen, Gemüse und/oder Gemüsezubereitungen) sowie geeignete Hilfs- und Trägerstoffe. Insbesondere sind hier (wie oben definierte) Aromakompositionen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Chili, Paprika, Kardamom, Muskat, Piment, Senf und Senf-Produkte), gebratenen, hefigen, gesottenen, fettigen, salzigen und/oder scharfen Aromaeindruck verursachen und somit vorteilhafterweise den würzigen Eindruck verstärken können. In der Regel enthalten die Aromakompositionen mehr als einen der genannten Inhaltsstoffe.

Gemäß einer bevorzugten Ausgestaltung der vorliegenden Erfindung umfasst eine erfindungsgemäße Zusammensetzung, (gebrauchs- oder verzehrfertige) Zubereitung oder Halbfertigware (wie jeweils oben beschrieben) zusätzlich
(a) eine oder mehrere Substanz(en) zum Maskieren oder Vermindern eines unangenehmen Geschmackseindrucks
   und/oder
(b) eine oder mehrere Substanz(en) zur Verstärkung oder Erzeugung eines angenehmen Geschmackseindrucks (wobei eine solche Substanz keine erfindungsgemäßen Verbindungen der Formeln (I) und (II) oder Verbindungen der Formeln (III) und (IV) umfasst). Im Zusammenhang mit der vorliegenden Erfindung handelt es sich bei einem unangenehmen Geschmackseindruck insbesondere um einen bitteren, metallischen, kalkigen, sauren und/oder adstringierenden Geschmackseindruck; bei einem angenehmen Geschmackseindruck handelt es sich insbesondere um einen süßen, salzigen oder einen Umami-Geschmackseindruck. Die Substanzen gemäß (a) und (b) sind Aromastoffe und im Rahmen der vorliegenden Erfindung einer Aromakomposition im Sinne der obigen Definition zuzuordnen, insbesondere bei einer quantitativen Betrachtung.

Durch zusätzlich enthaltene (a) Substanzen zum Maskieren oder Vermindern eines unangenehmen Geschmackseindrucks können (sofern vorhanden) unangenehme Geschmackseindrücke, insbesondere oben genannte unangenehme Geschmackseindrücke, vorteilhafterweise maskiert oder vermindert werden, so dass der insbesondere durch die erfindungsgemäßen Verbindungen der Formeln (I) und/oder (II) aufgewertete Geschmackseindruck der erfindungsgemäßen Zusammensetzung, (gebrauchs- oder verzehrfertigen) Zubereitung oder Halbfertigware insgesamt als vom Verbraucher angenehmer und hochwertiger empfunden wird.

Durch zusätzliche (b) Substanz(en) zur Verstärkung oder Erzeugung eines angenehmen Geschmackseindrucks (wie oben beschrieben) kann vorteilhafterweise insbesondere eine (additive, im Einzelfall sogar synergistische) Verstärkung des durch die erfindungsgemäßen Verbindungen der Formeln (I) und/oder (II) (bzw. durch erfindungsgemäße Mischungen oder Zusammensetzungen) erreichten Geschmackseindrucks, insbesondere des Umami-Geschmacks, erreicht werden.

Diese zusätzlichen Substanzen gemäß (a) oder (b) können aus der folgenden Liste ausgewählt werden, wobei sie jedoch nicht auf diese Auswahl beschränkt sind: Nucleotide (z.B. Adenosin-5'-monophosphat, Cytidin-5'-monophosphat, Inosin-5'-monophosphat, Guanosin-5'-monophosphat) oder deren pharmazeutisch akzeptable Salze, Lactisole, Hydroxyflavanone (z.B. Eriodictyol, Homoeriodictyol oder deren Natriumsalze), insbesondere gemäß EP 1 258 200, Hydroxybenzoesäureamide (z. B. 2,4-Dihydroxybenzoesäurevanillylamid, 4-Hydroxybenzoesäurevanillyiamid), Gemische von Molkeproteinen mit Lecithinen, Hefeextrakte, Pflanzenhydrolysate, pulverisierte Gemüse (z.B. Zwiebelpulver, Tomatenpulver), Pflanzenextrakte (z.B. von Liebstöckel oder von Pilzen wie Shütake), Meeralgen und Mineralsalzmischungen. Dabei können erfindungsgemäße Zusammensetzungen, Zubereitungen bzw. Halbfertigwaren eine oder mehrere dieser Substanzen enthalten.

Weitere vorteilhafte (geschmacksmodulierende) Aroma- und/oder Geschmackstoffe, die in einer erfindungsgemäßen Zusammensetzung, Zubereitung oder Halbfertigware enthalten sein können, sind vorzugsweise ausgewählt aus der Gruppe bestehend aus 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxybenzoesäureamiden, wie zum Beispiel 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N-*(4-hydroxy-3-methoxybenzyl)-amid, 2,4-Dihydroxybenzoesäure-*N*-(4-hydroxy-3-methoxybenzyl)amid-Mono-natriumsalz, 2,4-Dihydroxybenzoesäure-*N-*2-(4-hydroxy-3-methoxyphenyl)-ethyl-amid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamid (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl) ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); γ-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird) und Divanillinen (insbesondere Divanillin wie beschrieben in WO 2004/078302, die bezüglich der darin offenbarten entsprechenden Verbindungen auf dem Wege der Verweisung Bestandteil dieser Anmeldung wird); Bicyclo[4.1.0]heptan-7-carbonsäureamide, insbesondere solche wie beschrieben in WO 2008/046895, die bezüglich der darin offenbarten entsprechenden Verbindungen auf dem Wege der Verweisung Bestandteil dieser Anmeldung wird; Cyclopropancarbonsäure(3-methyl-cyclohexyl)amide, insbesondere solche wie beschrieben in US-Provisional 60/916,589 vom 08.05.2007 sowie den darauf basierenden Dokumenten (Symrise), die bezüglich der darin offenbarten entsprechenden Verbindungen auf dem Wege der Verweisung Bestandteil dieser Anmeldung wird; Aromatische *Neo-*Menthylamide, insbesondere solche wie beschrieben in US-Provisional Application 60/984,023 vom 31.10.2007 sowie den darauf basierenden Dokumenten (Symrise), die bezüglich der darin offenbarten entsprechenden Verbindungen auf dem Wege der Verweisung Bestandteil dieser Anmeldung wird; *Neo*-Menthylderivate, insbesondere solche wie beschrieben in der US-Provisional Application 61/061,273 vom 13.06.2008 sowie den darauf basierenden Dokumenten (Symrise), die bezüglich der darin offenbarten entsprechenden Verbindungen auf dem Wege der Verweisung Bestandteil dieser Anmeldung wird.

Gemäß einer weiteren Ausgestaltung der vorliegenden Erfindung umfasst eine jeweils wie oben beschriebene erfindungsgemäße Zusammensetzung, gebrauchs- oder verzehrfertige Zubereitung oder Halbfertigware insbesondere zusätzlich einen oder mehrere süßverstärkende Stoffe. Insbesondere werden die erfindungsgemäßen Verbindungen der Formeln (I) oder (II) bzw. die erfindungsgemäßen Mischungen (wie oben beschrieben) hierbei in Kombination mit zumindest einem süßverstärkenden Stoff eingesetzt, insbesondere mit einer oder mehreren Verbindungen gemäß WO 2007/014879 A1 oder WO 2007/107596 A1, speziell zusammen mit Hesperetin und/oder Phloretin. Hierdurch wird vorteilhafterweise eine Verstärkung und eine Vertiefung sowie Abrundung des Geschmacksprofils, insbesondere des würzigen und/oder salzigen Geschmacks der Zusammensetzung, Zubereitung oder Halbfertigware erreicht. Für Halbfertigwaren liegt der Gesamtanteil an Hesperetin und/oder Phloretin hierbei vorzugsweise im Bereich von 10 bis 100000 ppm, bezogen auf das Gesamtgewicht der Halbfertigware, während im verzehrfertigen Lebensmittel der Gesamtanteil an Hesperetin und/oder Phloretin bezogen auf das Gesamtgewicht des Lebensmittels vorzugsweise im Bereich von 1 bis 400 ppm, bevorzugt im Bereich von 5 bis 200 ppm liegt.

Vorzugsweise sind in den erfindungsgemäßen Zusammensetzungen, Zubereitungen bzw. Halbfertigwaren zusätzlich ein oder mehrere süßverstärkende Stoffe und ein oder mehrere weitere Geschmacksstoffe, die einen trigeminalen Reiz (tingling, kribbeln, scharf, kühlend etc.) bewirken, enthalten. Insbesondere bei der Kombination der erfindungsgemäßen Verbindungen der Formeln (I) oder (II) bzw. der entsprechenden erfindungsgemäßen Mischungen mit Hesperetin und/oder Phloretin aber auch mit cisund/oder trans-Pellitorin (siehe WO 2004/000787 bzw. WO 2004/043906) wird ein weiter verbessertes und vom Konsumenten bevorzugtes Geschmacksprofil erreicht. Dabei liegt der Gesamtanteil an cis- und/oder trans-Pellitorin in diesen Zusammensetzungen bzw. Zubereitungen oder Halbfertigwaren vorzugsweise im Bereich von 0,1 bis 500 ppm, bevorzugt im Bereich von 5 bis 100 ppm, bezogen auf das Gesamtgewicht der Zusammensetzung, Zubereitung oder Halbfertigware.

Aus dem vorstehenden Text ergibt sich, dass ein weiterer Aspekt der vorliegenden Erfindung auch ein Verfahren zum Erzeugen, Vermitteln, Modifizieren und/oder Verstärken eines Geschmacks, vorzugsweise eines Umami-Geschmacks, in einer der Ernährung, der Mundpflege oder dem Genuss dienenden (i) gebrauchs- oder verzehrfertigen Zubereitung oder (ii) Halbfertigware betrifft. Ein solches erfindungsgemäßes Verfahren umfasst den folgenden Schritt:
- Vermischen
   (A) einer geschmacklich wirksamen Menge einer erfindungsgemäßen Verbindung der Formel (I) oder (II) oder einer erfindungsgemäßen Mischung (wie oben beschrieben) oder
   (B) einer erfindungsgemäßen Zusammensetzung (wie oben beschrieben)
   mit einem oder mehreren weiteren Bestandteilen der (i) verzehrfertigen Zubereitung oder der (ii) Halbfertigware;
   oder
- Applizieren
   (A) einer geschmacklich wirksamen Menge einer erfindungsgemäßen Verbindung der Formel (I) oder (II) oder einer erfindungsgemäßen Mischung (wie oben beschrieben) oder
   (B) einer erfindungsgemäßen Zusammensetzung (wie oben beschrieben) auf einen oder mehrere weitere Bestandteile der (i) verzehrfertigen Zubereitung oder der (ii) Halbfertigware;
   oder
- Einbetten
   (A) einer geschmacklich wirksamen Menge einer erfindungsgemäßen Verbindung der Formel (I) oder (II) oder einer erfindungsgemäßen Mischung (wie oben beschrieben)
      oder
   (B) einer erfindungsgemäßen Zusammensetzung (wie oben beschrieben) in ein Hüll- oder Matrixmaterial.

Dabei gilt hinsichtlich bevorzugter erfindungsgemäßer Verbindungen und Mischungen das oben Gesagte entsprechend.

Nachfolgend wird die Erfindung anhand von Beispielen näher erläutert. Sofern nicht anders angegeben, beziehen sich alle Angaben auf das Gewicht.

### Beispiele:

### Allgemeine Arbeitsvorschrift (AAV1): Umsetzung mit Oxalsäuremonoesterchloriden

Die Lösung eines Amins oder Alkohols in DMC (Dimethylcarbonat) wird mit 1.0 bis 3.5 Äquivalenten Triethylamin versetzt und auf 0 bis 25 °C abgekühlt. Dann werden langsam 0.9 bis1.2 Äquivalente des entsprechenden Oxalsäuremonoesterchlorids zugetropft. Die Reaktion wird auf RT (Raumtemperatur) erwärmt und rührt für 4 bis12 h nach. Anschließend wird mit Dichlormethan verdünnt und nacheinander mit Wasser, 10%iger HCl und 5%iger NaOH gewaschen. Nach Trocknen über Natriumsulfat und anschließender Entfernung des Lösungsmittels am Rotationsverdampfer erfolgt die Aufreinigung entweder durch Chromatographie oder Destillation.

### Allgemeine Arbeitsvorschrift (AAV2): Umsetzung mit primären Aminen

Zu einer Lösung der nach AAV1 dargestellten Oxalamidmethylester in 5 bis 10 ml/mmol Diethylether werden 1.0 bis 1.5 Äquivalente einer kommerziell erhältlichen Lösung aus Ethyl- oder Methylamin in Wasser langsam zugetropft. Anschließend rührt die Reaktion für 8 Stunden bei Raumtemperatur. Nach Entfernung des Lösungsmittels am Rotationsverdampfer erfolgt die Aufreinigung entweder durch Chromatographie und/oder Kristallisation.

### Allgemeine Arbeitsvorschrift (AAV3): Umsetzung mit Oxalylchlorid

Zu der Lösung eines Amins sowie 3.0 bis 3.5 Äquivalenten Triethylamin in DMC werden unter Kühlung langsam 0.4 bis 0.5 Äquivalente Oxalylchlorid zugetropft. Die Reaktion wird auf RT erwärmt und rührt für 16 h nach. Anschließend wird mit Dichlormethan verdünnt und mit Wasser gewaschen. Nach Trocknen über Natriumsulfat und anschließender Entfernung des Lösungsmittels erfolgt die Aufreinigung durch Umkristallisation in Diethylether.

Im Folgenden werden ausgewählte gemäß den vorangehend beschriebenen allgemeinen Arbeitsvorschriften hergestellte Substanzen vorgestellt. Die Geschmacksprofile wurden jeweils durch Bestimmung des Geschmackseindrucks der jeweiligen Substanz als Reinstoff in Salz- oder Zuckerlösung bestimmt und weichen daher von den in Tabelle 1 und Fig.1 dargestellten Geschmackseindrücken (in Rindfleischextrakten) ab.

### Synthesebeispiel 1: N-((E)-3,7-Dimethyl-octa-2,6-dienyl)-oxalsäureamid-ethyl-ester, Verbindung der Formel (6)

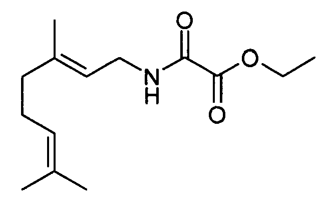

Die genannte Substanz wird nach **AAV 1** hergestellt, indem man Geranylamin und Oxalsäuremonoethylesterchlorid miteinander umsetzt und das Produkt säulenchromatographisch (Pentan/Diethylether = 2/1) aufreinigt.

### Analysendaten:

**¹H-NMR (400 MHz, CDCl₃):** 1.39 (t, *J* = 7.1 Hz, 3H); 1.60 (m, 3H); 1.69 (m, 6H); 2.00 - 2.13 (m, 4H); 3.94 (m, 2H); 4.35 (q, *J* = 7.1 Hz, 2H); 5.07 (tq, *J* = 1.3, 6.8 Hz, 1H); 5.21 (tq, *J* = 1.3, 7.2 Hz, 1H); 7.02 (bs, 1H) ppm.
**¹³C-NMR (100 MHz, CDCl₃):** 14.0 (CH₃); 16.4 (CH₃); 17.7 (CH₃); 25.7 (CH₃); 26.3 (CH₂); 37.8 (CH₂); 39.4 (CH₂); 63.2 (CH₂); 118.4 (CH); 123.7 (CH); 131.9 (C); 141.3 (C); 156.3 (C=O); 160.8 (C=O) ppm.
**Massenspektrum (EI):** m/z (%) = 253 (M˙⁺, 4); 180 (14); 156 (16); 136 (54); 131 (11); 123 (13); 121 (26); 118 (19); 112 (42); 110 (16); 93 (42); 92 (10); 90 (13); 81 (22); 80 (14); 69 (100); 68 (75); 67 (30); 53 (12); 41 (67); 29 (43); 27 (10).
**Geschmacksprofil:** krautig, muffig, chemisch.

### Synthesebeispiel 2: N-((E)-3,7-Dimethyl-octa-2,6-dienyl)-oxalsäureamid-methyl-ester, erfindungsgemäße Verbindung der Formel (I)

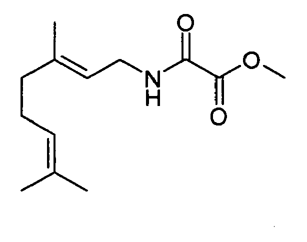

Die genannte Substanz wird nach **AAV 1** hergestellt, indem man Geranylamin und Oxalsäuremonomethylesterchlorid miteinander umsetzt und das Produkt säulenchromatographisch (Pentan/Diethylether = 1/1) aufreinigt.

### Analysendaten:

**¹H-NMR (400 MHz, CDCl₃):** 1.60 (m, 3H); 1.69 (m, 6H); 1.98 - 2.13 (m, 4H); 3.90 (s, 3H); 3.94 (m, 2H); 5.07 (m, 1H); 5.21 (tq, *J* = 1.3, 7.2 Hz, 1H); 7.02 (bs, 1H) ppm.
**¹³C-NMR (100 MHz, CDCl₃):** 16.4 (CH₃); 17.7 (CH₃); 25.7 (CH₃); 26.3 (CH₂); 37.8 (CH₂); 39.4 (CH₂); 53.6 (CH₃); 118.3 (CH); 123.7 (CH); 131.9 (C); 141.4 (C); 156.0 (C=O); 161.3 (C=O) ppm.
**Massenspektrum (EI):** m/z (%) = 239 (M˙⁺, 3); 180 (11); 137 (11); 136 (57); 123 (14); 121 (24); 117 (10); 112 (40); 111 (13); 104 (21); 93 (39); 92 (11); 83 (12); 82 (10); 81 (24); 80 (14); 69 (100); 68 (72); 67 (29); 59 (17); 55 (11); 53 (15); 42 (12); 41(72); 39 (10).
**Geschmacksprofil:** süß, Fülle, kribbeln, Umami.

### Synthesebeispiel 3: N-((Z)-3,7-Dimethyl-octa-2,6-dienyl)-oxalsäureamid-methyl-ester, erfindungsgemäße Verbindung der Formel (III)

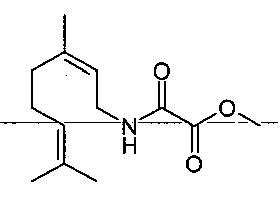

Die genannte Substanz wird nach **AAV 1** hergestellt, indem man Nerylamin und Oxalsäuremonomethylesterchlorid miteinander umsetzt und das Produkt säulenchromatographisch (Pentan/Diethylether = 3/2) aufreinigt.

### Analysendaten:

**¹H-NMR (400 MHz, CDCl₃):** 1.61 (m, 3H); 1.69 (m, 3H); 1.75 (dd, *J* = 1.1, 7.5 Hz, 3H); 2.07 - 2.11 zHz(m, 4H); 3.89 (s, 3H); 3.89 - 3.94 (m, 2H); 5.08 (m, 1 H); 5.22 (tq, *J* = 1.4, 7.3 Hz, 1H); 6.97 (bs, 1H) ppm.
**¹³C-NMR (100 MHz, CDCl₃):** 17.7 (CH₃); 23.3 (CH₃); 25.7 (CH₃); 26.4 (CH₂); 32.0 (CH₂); 37.6 (CH₂); 53.6 (CH₃); 119.2 (CH); 123.4 (CH); 132.4 (C); 141.4 (C); 156.0 (C=O); 161.2 (C=O) ppm.
**Massenspektrum (EI)**: m/z (%) = 239 (M˙⁺, 9); 180 (10); 137 (10); 136 (63); 123 (12); 121 (33); 112 (35); 111 (11); 110 (15); 104 (22); 93 (56); 92 (11); 81 (32); 80 (21); 69 (100); 68 (85); 67 (31); 59 (14); 53 (15); 43 (10); 42 (13); 41 (99); 39 (12).
**Geschmacksprofil:** muffig, bitter, pappig.

### Synthesebeispiel 4: Oxalsäure (E)-3,7-dimethyl-octa-2,6-dienyl-ester-methyl-ester

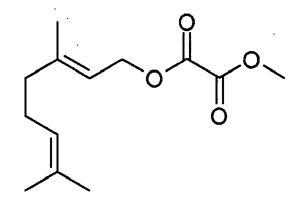

Die genannte Substanz wird nach **AAV 1** hergestellt, indem man Geraniol und Oxalsäuremonomethylesterchlorid miteinander umsetzt und das Produkt destillativ aufreinigt.

### Analysendaten:

**¹H-NMR (400 MHz, CDCl₃):** 1.60 (d, *J* = 0.8 Hz, 3H); 1.68 (d, *J* = 1.1 Hz, 3H); 1.74 (d, *J* = 1.34 Hz, 3H); 2.03 - 2.15 zHz(m, 4H); 3.90 (s, 3H); 4.81 (dd, *J* = 0.5, 7.3 Hz, 2H); 5.07 (m, 1H); 5.22 (tq, *J* = 1.3, 7.3 Hz, 1H) ppm.
**¹³C-NMR (100 MHz, CDCl₃):** 16.6 (CH₃); 17.7 (CH₃); 25.7 (CH₃); 26.2 (CH₂); 39.6 (CH₂); 53.5 (CH₃); 63.9 (CH₂); 116.7 (CH); 123.6 (CH); 132.0 (C); 144.6 (C); 157.6 (C=O); 158.3 (C=O) ppm.
**Massenspektrum (EI):** m/z (%) = 136 (8); 121 (6); 93 (14); 81 (6); 69 (100); 68 (29); 67 (12); 53 (5); 41 (37); 39 (6).
**Geschmacksprofil:** nur Zwischenprodukt, keine Geschmacksbeschreibung erfolgt.

### Synthesebeispiel 5: N,N'-Bis-((E)-3,7-dimethyl-octa-2,6-dienyl)-oxalamid, Verbindung der Formel (1)

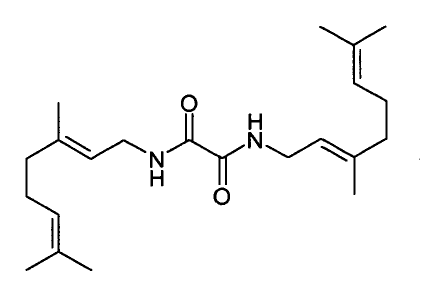

Die genannte Substanz wird nach **AAV 3** hergestellt, indem als Amin Geranylamin eingesetzt wurde.

### Analysendaten:

**¹H-NMR (400 MHz, CDCl₃):** 1.60 (m, 6H); 1.68 (m, 12H); 1.97 - 2.13 (m, 4H); 3.90 (m, 4H); 5.07 (m, 2H); 5.20 (tq, *J* = 1.3, 7.1 Hz, 2H); 7.37 (bs, 2H) ppm.
**¹³C-NMR (100 MHz, CDCl₃):** 16.4 (CH₃); 17.7 (CH₃); 25.7 (CH₃); 26.3 (CH₂); 37.5 (CH₂); 39.4 (CH₂); 118.6 (CH); 123.7 (CH); 131.9 (C); 140.9 (C); 159.6 (C=O) ppm.
**Massenspektrum (EI):** m/z (%) = 360 (M˙⁺, 7); 223 (13); 180 (10); 136 (25); 135 (36); 123 (12); 121 (17); 110 (5); 95 (9); 93 (33); 84 (16); 81 (35); 80 (11); 69 (100); 68 (21); 67 (14); 43 (6); 41 (52).
**Geschmacksprofil:** neutral, leicht scharf.

### Synthesebeispiel 6: N,N'-Bis-((Z)-3,7-dimethyl-octa-2,6-dienyl)-oxaiamid, Verbindung der Formel (2)

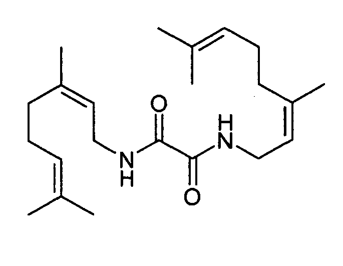

Die genannte Substanz wird nach **AAV 3** hergestellt, indem als Amin Nerylamin eingesetzt wurde.

### Analysendaten:

**¹H-NMR (400 MHz, CDCl₃):** 1.60 (m, 6H); 1.69 (m, 12H); 1.74 (dd, *J* = 1.0, 1.5 Hz, 4H); 2.06 - 2.12 (m, 4H); 3.88 (m, 4H); 5.09 (m, 2H); 5.20 (tq, *J* = 1.5, 7.2 Hz, 2H); 7.33 (bs, 2H) ppm.
**¹³C-NMR (100 MHz, CDCl₃):** 17.7 (CH₃); 23.3 (CH₃); 25.7 (CH₃); 26.4 (CH₂); 32.0 (CH₂); 37.4 (CH₂); 119.5 (CH); 123.5 (CH); 132.3 (C); 140.9 (C); 159.5 (C=O) ppm.
Massenspektrum (EI): m/z (%) = 360 (M˙⁺, 14); 223 (15); 155 (10); 137 (13); 136 (34); 135 (46); 121 (19); 107 (11); 95 (10); 93 (54); 92 (10); 84 (14); 81 (44); 80 (18); 69 (100); 41 (52).
**Geschmacksprofil:** neutral, leichtes Brennen.

### Synthesebeispiel 7: N-((E)-3,7-Dimethyl-octa-2,6-dienyl)-N'-methyl-oxalamid, erfindungsgemäße Verbindung der Formel (II)

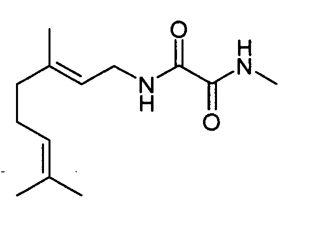

Die genannte Substanz wird nach **AAV 2** hergestellt, indem den aus Synthesebeispiel 2 erhaltenen N-((*E*)-3,7-Dimethyl-octa-2,6-dienyl)-oxalsäureamid-methyl-ester (**I**) mit Methylamin umsetzt.

### Analysendaten:

**¹H-NMR (400 MHz, CDCl₃):** 1.62 (m, 3H); 1.70 (m, 6H); 1.98 - 2.13 (m, 4H); 2.92 (d, *J* = 5.3 Hz, 3H); 3.92 (m, 2H); 5.08 (m, 1 H); 5.22 (m, 1H); 7.36 (bs, 1H), 7.46 (bs, 1H) ppm.
**¹³C-NMR (100 MHz, CDCl₃):** 16.4 (CH₃); 17.7 (CH₃); 25.7 (CH₃); 26.2 (CH₃); 26.3 (CH₂); 37.5 (CH₂); 39.4 (CH₂); 118.5 (CH); 123.7 (CH); 131.9 (C); 141.0 (C); 159.5 (C=O); 160.6 (C=O) ppm.
**Massenspektrum (EI):** m/z (%) = 238 (M˙⁺, 3); 169 (20); 152 (11); 136 (40); 123 (13); 121 (20); 93 (29); 84 (47); 81 (12); 70 (12); 69 (100); 68 (82); 67 (26); 58 (39); 53 (12); 41 (69); 30 (12).
**Geschmacksprofil:** fleischig, Fülle, Metall, Umami.

### Synthesebeispiel 8: N-((Z)-3,7-Dimethyl-octa-2,6-dienyl)-N'-methyl-oxalamid, erfindungsgemäße Verbindung der Formel (IV)

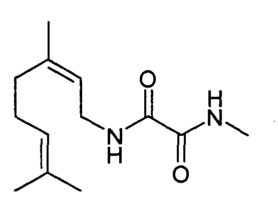

Die genannte Substanz wird nach **AAV 2** hergestellt, indem den aus Synthesebeispiel 3 erhaltenen N-((Z)-3,7-Dimethyl-octa-2,6-dienyl)-oxalsäureamid-methylester (**III**) mit Methylamin umsetzt.

### Analysendaten:

**¹H-NMR (400 MHz, CDCl₃):** 1.61 (m, 3H); 1.69 (m, 3H); 1.74 (m, 3H); 2.06 - 2.12 zHz(m, 4H); 2.90 (d, *J* = 5.3 Hz, 3H); 3.88 (m, 2H); 5.09 (m, 1H); 5.20 (m, 1H); 7.31 (bs, 1H); 7.44 (bs, 1H) ppm.
**¹³C-NMR (100 MHz, CDCl₃):** 17.7 (CH₃); 23.3 (CH₃); 25.7 (CH₃); 26.2 (CH₃); 26.4 (CH₂); 32.0 (CH₂); 37.4 (CH₂); 119.4 (CH); 123.5 (CH); 132.4 (C); 141.0 (C); 159.5 (C=O); 160.5 (C=O) ppm.
Massenspektrum (EI): m/z (%) = 238 (M˙⁺, 4); 180 (10); 169 (29); 152 (17); 136 (53); 123 (12); 121 (30); 103 (10); 94 (10); 93 (52); 84 (42); 82 (17); 81 (20); 80 (18); 79 (11); 70 (14); 69 (100); 68 (96); 67 (34); 58 (46); 55 (10); 53 (15); 43 (10); 42 (10); 41 (86); 39 (11); 30 (15).
**Geschmacksprofil:** sauer.

Die folgenden Strukturen sind weitere Beispiele aus dieser Substanzklasse, die entsprechend **AAV 1 und AAV 2 -** ausgehend von verschiedenen Aminen und Alkoholen - dargestellt und anschließend verkostet wurden.

**Tabelle 2**

| **Struktur** | **MS-daten** | **Geschmacksprofil** |
|---|---|---|
| 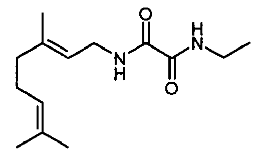 | **m/z** (%) = 252 (M˙⁺, 8); 183 (46); 136 (71); 121 (33); 93 (47); 84 (61); 69 (90); 68 (100); 44 (31); 41 (66). | neutral |
| 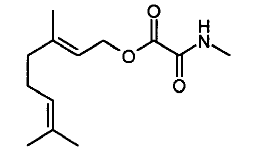 | **m/z** (%) = 121 (16); 94 (12); 93 (50); 81 (12); 69 (100); 68 (82); 67 (16); 58 (35); 41 (61); 39 (11). | seifig, blumig, parfümistisch |
| 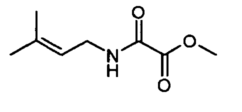 | **m/z** (%) = 171 (M˙⁺, 26); 112 (45); 111 (22); 83 (35); 69 (100); 68 (41); 67 (23); 43 (15); 41 (89); 39 (15). | Eigengeschmack, Lösungsmittel |
| 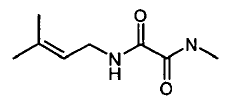 | **m/z** (%) = 170 (M˙⁺, 16); 84 (100); 70 (30); 69 (75); 68 (49); 67 (16); 58 (50); 42 (12); 41 (77); 39 (12). | neutral |
| 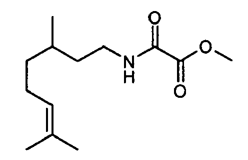 | **m/z** (%) = 241 (M˙⁺, 1); 182 (100); 117 (68); 104 (36); 95 (33); 82 (27); 81 (31); 69 (72); 55 (38); 41 (68). | neutral |
| 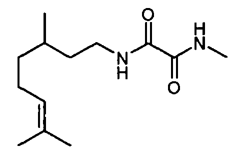 | **m/z** (%) = 240 (M˙⁺, 5); 182 (100); 95 (47); 82 (40); 81 (41); 69 (87); 58 (54); 55 (48); 41 (88); 30 (62). | fruchtig |
| 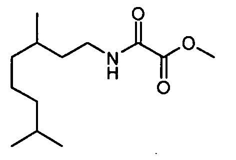 | **m/z** (%) = 243 (M˙⁺, 0.5); 184 (100); 130 (31); 117 (99.6); 104 (35); 71 (42); 57 (70); 55 (41); 43 (81); 41 (45). | fettig, bitter, metallisch |
| 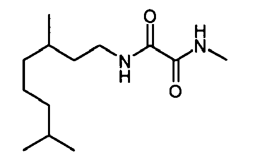 | **m/z** (%) = 242 (M˙⁺, 4); 184 (100); 116 (26); 71 (27); 58 (31); 57 (42); 55 (22); 43 (43); 41 (24); 30 (36). | neutral |

### Anwendungsbeispiele

Die folgenden Anwendungsbeispiele dienen zur Verdeutlichung der Erfindung, ohne diese dadurch einzuschränken. Sofern nicht anders angegeben, beziehen sich alle Angaben auf das Gewicht.

### Anwendungsbeispiel 1: Sprühgetrocknete Zusammensetzungen

**1.1**

| **Bestandteil** | **Anteil** |
|---|---|
| Mischung aus N-((*E*)-3,7-Dimethyl-octa-2,6-dienyl)-oxalsäureamid methyl ester (I) und *N*-((E)-3,7-Dimethyl-octa-2,6-dienyl-*N'-*methyloxalamid (II) | 4 g |
| Maltodextrin | 96 g |

**1.2**

| **Bestandteil** | **Anteil** |
|---|---|
| *N*-((E)-3,7-Dimethyl-octa-2,6-dienyl)-*N*-methyl-oxalamid **(II)** | 8 g |
| Maltodextrin | 92 g |

Die Bestandteile werden in einer Mischung aus Ethanol und demineralisiertem Wasser gelöst und anschließend sprühgetrocknet.

### Anwendungsbeispiel 2: Aromakomposition, nicht erfindungsgemäß

| **Inhaltsstoff** | **Anteil** |
|---|---|
| 10 Gew.-% Pellitorin in 1,2-Propylenglycol/Diethylmalonat | 0,25 g |
| Hesperetin | 2,50 g |
| Phloretin | 1,50 g |
| Propylenglycol | 95,75 g |

Die Aromakomposition wurde in nachfolgend beschriebenen Anwendungsbeispielen eingesetzt.

### Anwendungsbeispiel 3: Würzmittel

| **Teil** | **Bestandteil** | **Anteil** |
|---|---|---|
| A | Verbindung der Formel **(I)** | 0,04 g |
| | Natriumchlorid | 15 g |
| B | Senfsamenmehl | 5 g |
| | Senfaroma | 0,1 g |

Teil A wurde eingewogen. Es wurden 290 ml Wasser vorgelegt und unter Rühren Teil A zugegeben und gelöst. Die Lösung wird mit Wasser auf 1,84 kg verdünnt (pH 6,0) und anschließend gefriergetrocknet (Eutektischer Punkt: -15°C; Arbeitsvakuum: 0,52 mbar; Stellflächentemperatur: -5°C bis +25°C). Das Produkt wird mit Senfsamenmehl und dem Senfaroma aus Teil B gemischt und zu einem Würzmittel konfektioniert.

### Anwendungsbeispiel 4: Umami-Reaktionsaroma

| **Inhaltsstoff** | **Einsatz [g]** |
|---|---|
| L-Alanin | 41,0 |
| L-Asparaginsäure | 123,0 |
| Bernsteinsäure | 4,7 |
| Calciumchlorid Dihydrat | 7,0 |
| L-Cystein•HCl Monohydrat | 11,0 |
| Dikaliumphosphat | 6,0 |
| Fructose gemahlen | 1,0 |
| L-Isoleucin | 1,6 |
| Kaliumchlorid | 228,0 |
| L-Leucin | 1,6 |
| L-Lysin•HCl | 3,6 |
| Magnesiumchlorid Hexahydrat | 19,0 |
| Maltodextrin | 49,0 |
| L-Phenylalanin | 2,0 |
| L-Prolin | 74,0 |
| L-Serin | 6,5 |
| L-Threonin | 3,0 |
| L-Valin | 9,0 |
| Wasser | 389,0 |
| Verbindung der Formel **(II),** 20 Gew.-% in EtOH | 20,0 |

Alle Komponenten werden bei 40°C gemischt und anschließend bei 85°C für 10 Minuten erhitzt (Rückflussreaktion). Nach dem Abkühlen auf 40°C wird mit Kalilauge auf pH 5 eingestellt. Dieses Umami-Reaktionsaroma kann anstelle der reinen Verbindung **(II)** in die Bouillon-Zubereitungen C bzw. D des Anwendungsbeispiels 9 eingearbeitet werden, wobei in Zubereitung C 5 g und in Zubereitung D 13 g des Umami-Reaktionsaromas verwendet wurden.

### Anwendungsbeispiel 5: Verqleichsuntersuchung "Instantsuppe, Tvp Lauch-Creme"

A = Vergleichszubereitung
B, C, D = erfindungsgemäße Zubereitungen (Natriumglutamatfrei)

| **Bestandteil** | **A** | **B** | **C** | **D** |
|---|---|---|---|---|
| Kartoffelstärke | 20,00 g | 20,00 g | 20,00 g | 20,00 g |
| Fettpulver | 25,00 g | 25,00 g | 25,00 g | 25,00 g |
| Lactose | 20,00 g | 20,00 g | 20,00 g | 20,00 g |
| Maltodextrin | 11,73 g | 14,72 g | 14,71 g | 14,67 g |
| Kochsalz | 8,00 g | 8,00 g | 8,00 g | 8,00 g |
| Natriumglutamat | 3,00 g | - | - | - |
| Spinatpulver | 2,00 g | 2,00 g | 2,00 g | 2,00 g |
| Grünes Lauchpulver | 2,00 g | 2,00 g | 2,00 g | 2,00 g |
| Citronensäure, als Pulver | 0,30 g | 0,30 g | 0,30 g | 0,30 g |
| Gehärtetes Pflanzenfett | 3,00 g | 3,00 g | 3,00 g | 3,00 g |
| Gefriergetrockneter Lauch | 1,00 g | 1,00 g | 1,00 g | 1,00 g |
| Huhnaroma | 1,00 g | 1,00 g | 1,00 g | 1,00 g |
| Würzmischung Typ "grüner Lauch", Pulver | 2,00 g | 2,00 g | 2,00 g | 2,00 g |
| Würzmischung, Typ "gekochte Zwiebel" | 0,60 g | 0,60 g | 0,60 g | 0,60 g |
| Hefe-Würzmischung, Typ "Gemüsebrühe", Pulver | 0,30 g | 0,30 g | 0,30 g | 0,30 g |
| Curcuma-Extrakt | 0,07 g | 0,07 g | 0,07 g | 0,07 g |
| *N*-((E)-3,7-Dimethyl-octa-2,6-dienyl)-*N'*-methyloxalamid **(II)** | - | 0,01 g | 0,02 g | 0,06 g |

5 g der jeweiligen Pulvermischung wurden mit je 100 ml heißem Wasser aufgegossen, um eine verzehrfertige Suppe zu erhalten.
Bei der Verkostung durch ein Panel ausgebildeter Testpersonen wurden die Vergleichszubereitung A und die erfindungsgemäße Zubereitung C gleich bewertet. Bei der erfindungsgemäßen Zubereitung B wurden Umami-Geschmack (und Mundfülle) als wahrnehmbar, jedoch schwächer gegenüber den Zubereitungen A und C beschrieben. Die erfindungsgemäße Zubereitung D wurde hinsichtlich Umami-Geschmack (und Mundfülle) als sehr ausgeprägt und deutlich stärker als die Zubereitungen A und C bewertet.

### Anwendungsbeispiel 6: Vergleichsuntersuchung "Instantsuppe, Tvp Hühnersuppe mit Nudeln"

A = Vergleichszubereitung
B, C, D = erfindungsgemäße Zubereitungen (Natriumglutamat-frei)

| **Bestandteil** | **A** | **B** | **C** | **D** |
|---|---|---|---|---|
| Stärke | 16,0, g | 16,00 g | 16,00 g | 16,00 g |
| Kochsalz | 7,00 g | 7,00 g | 7,00 g | 7,00 g |
| Saccharose, raffiniert | 3,20 g | 3,20 g | 3,20 g | 3,20 g |
| Natriumglutamat | 3,20 g | - | - | - |
| Natriuminosinat / Natriumguanylat im Verhältnis 1:1 | 0,80 g | 0,80 g | 0,80 g | 0,80 g |
| Säurehydrolysiertes Pflanzenprotein | 8,00 g | 8,00 g | 8,00 g | 8,00 g |
| Fettpulver | 2,00 g | 2,00 g | 2,00 g | 2,00 g |
| Gemüsefett, sprühgetrocknet | 1,00 g | 1,00 g | 1,00 g | 1,00 g |
| Gefriergetrocknetes Hühnerfleisch, in Stückchen | 2,00 g | 2,00 g | 2,00 g | 2,00 g |
| Suppen-Nudeln | 32,00 g | 32,00 g | 32,00 g | 32,00 g |
| Maltodextrin | 12,16 g | 15,35 g | 15,34 g | 14,11 g |
| Chinesisches Gemüse, gefriergetrocknet | 4,60 g | 4,60 g | 4,60 g | 4,60 g |
| Huhnaroma | 8,00 g | 8,00 g | 8,00 g | 8,00 g |
| Lebensmittelfarbstoff Riboflavin | 0.04 g | 0.04 g | 0.04 g | 0.04 g |
| *N*-((E)-3,7-Dimethyl-octa-2,6-dienyl)-*N'*-methyl-oxalamid **(II)** | - | 0,01 g | 0,02 g | 0,05 g |
| Aromenkomposition nach Anwendungsbeispiel 2 | - | - | - | 1,20 g |

4,6 g der jeweiligen Pulvermischung wurden 10 Minuten in je 100 ml Wasser gekocht, um eine verzehrfertige Suppe zu erhalten.
Bei der Verkostung durch ein Panel ausgebildeter Testpersonen wurden die Vergleichszubereitung A und die erfindungsgemäße Zubereitung C gleich bewertet. Bei der erfindungsgemäßen Zubereitung B wurden Umami-Geschmack (und Mundfülle) als wahrnehmbar, jedoch schwächer gegenüber den Zubereitungen A und C beschrieben. Die erfindungsgemäße Zubereitung D wurde hinsichtlich Umami-Geschmack (und Mundfülle) als sehr ausgeprägt und deutlich stärker als die Zubereitungen A und C bewertet.

### Anwendungsbeispiel 7: Vergleichsuntersuchung "Würzmischung, Typ "Pfeffer""

A = Vergleichszubereitung
B, C, D = erfindungsgemäße Zubereitungen (Natriumglutamat-frei)

| **Bestandteil** | **A** | **B** | **C** | **D** |
|---|---|---|---|---|
| Milchprotein | 0,80 g | 0,80 g | 0,80 g | 0,80 g |
| Johannisbrotkernmehl | 2,00 g | 2,00 g | 2,00 g | 2,00 g |
| Maisstärke | 22,00 g | 27,98 g | 27,94 g | 27,88 g |
| Kochsalz | 14,00 g | 14,00 g | 14,00 g | 14,00 g |
| Paprikapulver | 13,00 g | 13,00 g | 13,00 g | 13,00 g |
| Tomatenpulver | 13,00 g | 13,00 g | 13,00 g | 13,00 g |
| Saccharose | 4,00 g | 4,00 g | 4,00 g | 4,00 g |
| Knoblauchpulver | 0,50 g | 0,50 g | 0,50 g | 0,50 g |
| Gehärtetes Pflanzenfett | 8,00 g | 8,00 g | 8,00 g | 8,00 g |
| Fettpulver | 11,00 g | 11,00 g | 11,00 g | 11,00 g |
| Natriumglutamat | 6,00 g | - | - | - |
| Lebensmittelfarbstoff Rote Bete und Paprika | 2,00 g | 2,00 g | 2,00 g | 2,00 g |
| Aroma Typ "Pfeffer" | 2,00 g | 2,00 g | 2,00 g | 2,00 g |
| Aroma Typ "Pizza" | 1,20 g | 1,20 g | 1,20 g | 1,20 g |
| Aroma Typ "Tomate" | 0,40 g | 0,40 g | 0,40 g | 0,40 g |
| Extrakt aus schwarzem Pfeffer | 0,10 g | 0,10 g | 0,10 g | 0,10 g |
| *N-*((E)-3,7-Dimethylocta-2,6-dienyl)-*N'*-methyl-oxalamid **(II)** | - | 0,02 g | 0,06 g | 0,10 g |

Jeweils 100 g Nackensteak vom Schwein wurden mit jeweils 1,7 g der Zubereitungen A, B, C und D gleichmäßig bestreut und gebraten. Bei der Verkostung durch ein Panel ausgebildeter Testpersonen wurden die Vergleichszubereitung A und die erfindungsgemäße Zubereitung C gleich bewertet. Bei der erfindungsgemäßen Zubereitung B wurden Umami-Geschmack (und Mundfülle) als wahrnehmbar, jedoch schwächer gegenüber den Zubereitungen A und C beschrieben. Die erfindungsgemäße Zubereitung D wurde hinsichtlich Umami-Geschmack (und Mundfülle) als sehr ausgeprägt und deutlich stärker als die Zubereitungen A und C bewertet.

### Anwendungsbeispiel 8: Vergleichsuntersuchung "Tomatenketchup"

A = Vergleichszubereitung
B = Vergleichszubereitung (Natriumglutamat-frei, Zucker-reduziert)
C = erfindungsgemäße Zubereitungen (Natriumglutamat-frei, Zucker-reduziert)

| **Bestandteil** | **A** | **B** | **C** |
|---|---|---|---|
| Natriumglutamat | 0,4 g | - | - |
| Kochsalz | 2,0 g | 2,0 g | 2,0 g |
| Stärke, Farinex WM 55 | 1,0 g | 1,0 g | 1,0 g |
| Sucrose | 12,0 g | 9,2 g | 9,2 g |
| Tomaten-Konzentrat 2-fach | 40,0 g | 40,0 g | 40,0 g |
| Glucosesirup 80 Brix | 18,0 g | 18,0 g | 18,0 g |
| Branntweinessig 10% | 7,0 g | 7,0 g | 7,0 g |
| Wasser | 19,6 g | 22,8 g | 22,2 g |
| Aromenkomposition nach Anwendungsbeispiel 2 | - | - | 0,4 g |
| 1%ige Lösung von *N*-((E)-3,7-Dimethyl-octa-2,6-dienyl)-oxalamid-methylester **(I)** in Propylenglycol | - | - | 0.2 g |

Die Inhaltsstoffe werden in der angegebenen Reihenfolge gemischt und das fertige Ketchup mit Hilfe eines Rührwerkes homogenisiert, in Flaschen abgefüllt und sterilisiert.

### Anwendungsbeispiel 9: Vergleichsuntersuchung "Bouillon"

A = Vergleichszubereitung
B = Vergleichszubereitung (Natriumglutamat-reduziert)
C = erfindungsgemäße Zubereitungen (Natriumglutamat-reduziert)
D = erfindungsgemäße Zubereitungen (Natriumglutamat-frei)

| **Bestandteil** | **A** | **B** | **C** | **D** |
|---|---|---|---|---|
| Fettpulver | 8,77 g | 8,77 g | 8,77 g | 8,77 g |
| Natriumglutamat | 8,77 g | 5 g | 5 g | - |
| Hefeextrakt Pulver | 12,28 g | 12,28 g | 12,28 g | 12,28 g |
| Kochsalz | 29,83 g | 29,83 g | 29,83 g | 29,83 g |
| Maltodextrin | 37,28 g | 41,05 g | 41,01 g | 45,95 g |
| Natürlicher Gemüseextrakt | 3,07 g | 3,07 g | 3,07 g | 3,07 g |
| *N*-((E)-3,7-Dimethyl-octa-2,6-dienyl)-*N'*-methyloxalamid **(II)** | - | - | 0,04 g | 0,10 g |

15 g der jeweiligen Pulvermischung wurden mit je 1000 ml heißem Wasser aufgegossen. Bei der Verkostung durch ein Panel ausgebildeter Testpersonen wurden die Vergleichszubereitung A und die erfindungsgemäße Natriumglutamat-reduzierte Zubereitung C bzw. Natriumglutamat-freie Zubereitung D sehr ähnlich bezeichnet. Insgesamt wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet.

### Anwendungsbeispiel 10: Verqleichsuntersuchung "Würzmischung für Kartoffel-chips"

A = Vergleichszubereitung
B = Vergleichszubereitung (Natriumglutamat-reduziert)
C = erfindungsgemäße Zubereitungen (Natriumglutamat-reduziert)
D = erfindungsgemäße Zubereitungen (Natriumglutamat-frei)

| **Bestandteil** | **A** | **B** | **C** | **D** |
|---|---|---|---|---|
| Natriumglutamat | 3,50 g | 2,00 g | 2,00 g | - |
| Käsepulver | 10,00 g | 10,00 g | 10,00 g | 10,00 g |
| Knoblauchpulver | 2,00 g | 2,00 g | 2,00 g | 2,00 g |
| Molkenpulver | 38,86 g | 40,36 g | 40,06 g | 41,76 g |
| Würzextraktöl | 0,20 g | 0,20 g | 0,20 g | 0,20 g |
| Paprikapulver | 9,80 g | 9,80 g | 9,80 g | 9,80 g |
| Kochsalz | 21,00 g | 21,00 g | 21,00 g | 21,00 g |
| Tomatenpulver | 9,00 g | 9,00 g | 9,00 g | 9,00 g |
| Trockenaroma | 2,50 g | 2,50 g | 2,50 g | 2,50 g |
| Siliciumdioxid | 0,02 g | 0,02 g | 0,02 g | 0,02 g |
| Pflanzenöl | 0,02 g | 0,02 g | 0,02 g | 0,02 g |
| Zwiebelpulver | 3,00 g | 3,00 g | 3,00 g | 3,00 g |
| Sahne Aromakonzentrat | 0,03 g | 0,03 g | 0,03 g | 0,03 g |
| Käse Aroma | 0,03 g | 0,03 g | 0,03 g | 0,03 g |
| Tomaten Aromakonzentrat | 0,04 g | 0,04 g | 0,04 g | 0,04 g |
| Sprühgetrocknete Zusammensetzung gemäß Beispiel 1.1 | - | - | 0,30 g | 0,60 g |

6 g der Würzmischung wurden auf 94 g Kartoffelchips aufgezogen. Bei der Verkostung durch ein Panel ausgebildeter Testpersonen wurden die Vergleichszubereitung A und die erfindungsgemäße Natriumglutamat-reduzierte Zubereitung C bzw. Natriumglutamat-freie Zubereitung D sehr ähnlich bezeichnet. Insgesamt wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet.

### Anwendungsbeispiel 11: Vergleichsuntersuchung "Weiße Soße"

A = Vergleichszubereitung
B = Vergleichszubereitung (Natriumglutamat-reduziert)
C = erfindungsgemäße Zubereitungen (Natriumglutamat-reduziert)
D = erfindungsgemäße Zubereitungen (Natriumglutamat-frei)

| **Bestandteil** | **A** | **B** | **C** | **D** |
|---|---|---|---|---|
| Maltodextrin | 25,98 g | 27,18 g | 27,03 g | 27,75 g |
| Kochsalz | 7,50 g | 7,50 g | 7,50 g | 7,50 g |
| Natriumglutamat | 2,00 g | 0,80 g | 0,80 g | - |
| Pflanzenfett | 5,00 g | 5,00 g | 5,00 g | 5,00 g |
| Pfeffer, weiß | 0,02 g | 0,02 g | 0,02 g | 0,02 g |
| Zwiebelpulver | 1,50 g | 1,50 g | 1,50 g | 1,50 g |
| vorverkleisterte Maisstärke | 30,00 g | 30,00 g | 30,00 g | 30,00 g |
| Fettpulver | 28,00 g | 28,00 g | 28,00 g | 28,00 g |
| Sprühgetrocknete Zusammensetzung gemäß Beispiel **1.2** | - | - | 0,15 g | 0,23 g |

90 g der Soßenmischung wurden mit 1000 ml heißem Wasser aufgegossen und mit dem Schneebesen kräftig verrührt. Bei der Verkostung durch ein Panel ausgebildeter Testpersonen wurden die Vergleichszubereitung A und die erfindungsgemäße Natriumglutamat-reduzierte Zubereitung C bzw. Natriumglutamat-freie Zubereitung D sehr ähnlich bezeichnet. Insgesamt wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet.

### Anwendungsbeispiel 12: Vergleichsuntersuchung "Braune Soße"

A = Vergleichszubereitung
B = Vergleichszubereitung (Natriumglutamat-reduziert)
C = erfindungsgemäße Zubereitungen (Natriumglutamat-reduziert)
D = erfindungsgemäße Zubereitungen (Natriumglutamat-frei)

| **Bestandteil** | **A** | **B** | **C** | **D** |
|---|---|---|---|---|
| Stärke | 40,00 g | 40,70 g | 40,63 g | 41,77 g |
| Maltodextrin | 33,10 g | 33,10 g | 33,10 g | 33,10 g |
| Kochsalz | 6,00 g | 6,00 g | 6,00 g | 6,00 g |
| Zuckerkulör, sprühgetrocknet | 5,00 g | 5,00 g | 5,00 g | 5,00 g |
| Hefeextraktpulver | 3,00 g | 3,00 g | 3,00 g | 3,00 g |
| Natriumglutamat | 2,00 g | 1,30 g | 1,30 g | - |
| Zucker | 0,50 g | 0,50 g | 0,50 g | 0,50 g |
| Fettpulver | 5,00 g | 5,00 g | 5,00 g | 5,00 g |
| Tomatenpulver | 3,00 g | 3,00 g | 3,00 g | 3,00 g |
| Natürlicher Gemüseextrakt | 1,00 g | 1,00 g | 1,00 g | 1,00 g |
| Zwiebelextrakt | 0,30 g | 0,30 g | 0,30 g | 0,30 g |
| Pfefferextrakt | 0,10 g | 0,10 g | 0,10 g | 0,10 g |
| Trockenaroma | 1,00 g | 1,00 g | 1,00 g | 1,00 g |
| Sprühgetrocknete Zusammensetzung gemäß Beispiel 1.2 | - | - | 0,07 g | 0,23 g |

90 g der Soßenmischung wurden mit 1000 ml heißem Wasser aufgegossen und mit dem Schneebesen kräftig verrührt. Bei der Verkostung durch ein Panel ausgebildeter Testpersonen wurden die Vergleichszubereitung A und die erfindungsgemäße Natriumglutamat-reduzierte Zubereitung C bzw. Natriumglutamat-freie Zubereitung D sehr ähnlich bezeichnet. Insgesamt wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet.

### Anwendungsbeispiel 13: Vergleichsuntersuchung "Tomatensuppe"

A = Vergleichszubereitung
B = Vergleichszubereitung (Natriumglutamat-reduziert)
C = erfindungsgemäße Zubereitungen (Natriumglutamat-reduziert)
D = erfindungsgemäße Zubereitungen (Natriumglutamat-frei)

| **Bestandteil** | **A** | **B** | **C** | **D** |
|---|---|---|---|---|
| Wasser | 50,650 g | 50,80 g | 50,794 g | 51,030 g |
| Pflanzenöl | 5,500 g | 5,500 g | 5,500 g | 5,500 g |
| Tomatenpaste | 24,000 g | 24,000 g | 24,000 g | 24,000 g |
| Sahne | 1,050 g | 1,050 g | 1,050 g | 1,050 g |
| Zucker | 2,000 g | 2,000 g | 2,000 g | 2,000 g |
| Kochsalz | 1,700 g | 1,700 g | 1,700 g | 1,700 g |
| Natriumglutamat | 0,400 g | 0,250 g | 0,250 g | - |
| Weizenmehl | 5,500 g | 5,500 g | 5,500 g | 5,500 g |
| Stärke | 1,200 g | 1,200 g | 1,200 g | 1,200 g |
| gewürfelte Tomaten | 8,000 g | 8,000 g | 8,000 g | 8,000 g |
| Sprühgetrocknete Zusammensetzung gemäß Beispiel 1.2 | - | - | 0,006 g | 0,020 g |

Die festen Bestandteile wurden eingewogen, gemischt und dem Wasser hinzugefügt. Das Pflanzenöl wurde zudosiert und die Tomatenpaste hinzugegeben. Die Mischung wurde unter Rühren aufgekocht. Bei der Verkostung durch ein Panel ausgebildeter Testpersonen wurden die Vergleichszubereitung A und die erfindungsgemäße Natriumglutamat-reduzierte Zubereitung C bzw. Natriumglutamat-freie Zubereitung D sehr ähnlich bezeichnet. Insgesamt wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet.

### Anwendungsbeispiel 14: Vergleichsuntersuchung "Anwendung in einem zuckerfreien Kaugummi"

| **Teil** | **Inhaltsstoff** | **Einsatz in Gew.-%** |
|---|---|---|
| A | Kaugummibase, Company "Jagum T" | 29,991 |
| B | Sorbit, pulverisiert | 39,000 |
| | Isomalt® (Palatinit GmbH) | 9,500 |
| | Xylit | 2,000 |
| | Mannit | 3,000 |
| | Aspartam® | 0,100 |
| | Acesulfam® K | 0,100 |
| | Emulgum® (Colloides Naturels, Inc.) | 0,300 |
| C | Sorbitol, 70 % | 14,000 |
| | Glycerin | 1,000 |
| D | Aromakomposition, entsprechend Anwendungsbeispiel **2** | 1,000 |
| | *N*-((E)-3,7-Dimethyl-octa-2,6-dienyl)-*N*'-methyloxalamid **(II)** | 0,009 |

Teile A bis D werden gemischt und intensiv geknetet. Die Rohmasse kann z.B. in Form von dünnen Streifen zu verzehrfertigen Kaugummis verarbeitet werden.

### Anwendungsbeispiel 15: Vergleichsuntersuchung "Anwendung in einem Grünteegetränk"

| **Inhaltsstoff** | **Einsatz in Gew.%** |
|---|---|
| Grünteekonzentrat | 18,000 |
| 1%ige Lösung von *N*-((E)-3,7-Dimethyl-octa-2,6-dienyl)-oxalamidmethylester **(I)** in Propylenglycol | 0,008 |
| entmineralisiertes Wasser | 81,992 |

Das Grünteekonzentrat wird mit der 1% igen Lösung von *N*-((E)-3,7-Dimethyl-octa-2,6-dienyl)-oxalamid-methylester **(I)** in Propylenglycol vermischt. Anschließend wird mit entmineralisiertem Wasser aufgefüllt und erneut gründlich durchmischt. Dann wird das Produkt gefiltert, verbrauchsfertig verpackt und bei 118°C sterilisiert. Der Geschmack wird durch ein Panel ausgebildeter Testpersonen als deutlich bevorzugt gegenüber der nicht aromatisierten Grünteebase bewertet.

### Anwendungsbeispiel 16: Vergleichsuntersuchung "Rindfleischwürzmischung für (Fertig)-Nudeln"

| **Inhaltsstoff** | **Gew.%** |
|---|---|
| Rindsfettaroma | 5,00 |
| Zuckercouleur | 3,00 |
| Zitronensäure (wasserfrei) | 0,40 |
| Schnittlauch (entwässert) | 2,00 |
| Knoblauchpulver | 3,50 |
| Maltodextrin (ex Tapoica) | 10,25 |
| Mononatriumglutamat | 15,00 |
| Zwiebelpulver | 5,00 |
| Ribotide | 0,80 |
| Natriumchlorid | 45,65 |
| Zucker | 2,80 |
| Süßmolkepulver | 6,50 |
| 1%ige Lösung von *N*-((E)-3,7-Dimethylocta-2,6-dienyl)-*N'*-methyloxalamid **(II)** in Propylenglycol | 0,10 |

Alle Inhaltsstoffe werden vermischt bis sich eine homogene Mischung ergibt.

### Anwendungsbeispiel 17: Vergleichsuntersuchung "(Fertig)-Nudeln"

| **Teil** | **Inhaltsstoff** | **Gew.-%** |
|---|---|---|
| A | Weizenmehl | 62,00 |
| | Kartoffelstärke | 10,90 |
| B | Salz | 1,10 |
| | Guarkernmehl | 0,06 |
| | Natriumcarbonat | 0,07 |
| | Kaliumcarbonat | 0,25 |
| | Na₂H₂P₂O₇ | 0,07 |
| | 1%ige Lösung von *N*-((E)-3,7-Dimethylocta-2,6-dienyl)-oxalamidmethylester **(I)** in Propylenglycol | 0,10 |
| C | Wasser | 25,45 |

Eine Suspension der Zutaten B in Wasser (C) wird zu einer Mischung der Zutaten A gegeben und zu einem Teig geknetet. Nachdem der Teig für ca. 5 Minuten geruht hat, wird dieser mit Hilfe einer Nudelmaschine zu Platten verarbeitet, die in einem letzten Arbeitsschritt in eine übliche Form zurechtgeschnitten werden. Die Nudeln sind nach einer Kochzeit von 3 Minuten verzehrfertig und werden mit 8 g der Rindfleischwürzmischung (Anwendungsbeispiel 16) angerichtet.

## Patentansprüche

1. Verbindung der Formel (I) oder (II)

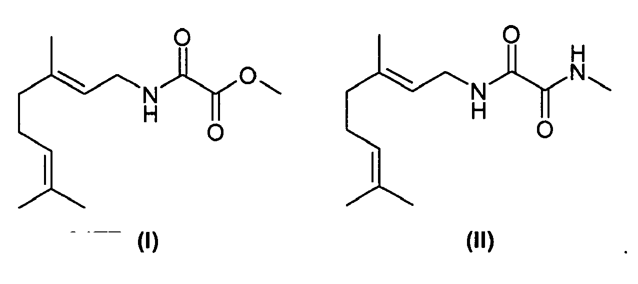

2. Mischung, insbesondere Geschmacksstoffmischung, umfassend eine Verbindung der Formel (I) und/oder eine Verbindung der Formel (II) oder bestehend aus einer Verbindung der Formel (I) und einer Verbindung der Formel (II) nach Anspruch 1.

3. Mischung, insbesondere Geschmacksstoffmischung, umfassend oder bestehend aus
- einer Verbindung der Formel (I) nach Anspruch 1 sowie einer Verbindung der Formel (III) 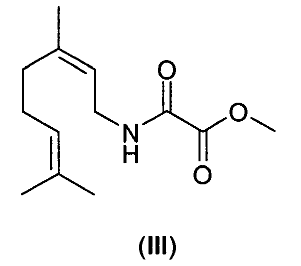 oder
- einer Verbindung der Formel (II) nach Anspruch 1 sowie einer Verbindung der Formel (IV) 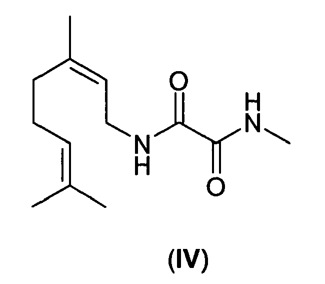 oder
- sämtlichen Verbindungen der Formeln (I), (II), (III) und (IV).

4. Mischung nach Anspruch 3, wobei das Gewichtsverhältnis von der Gesamtmenge an Verbindungen der Formeln (I) und (II) zu der Gesamtmenge an Verbindungen der Formeln (III) und (IV) 85:15 oder mehr, vorzugsweise 90:10 oder mehr, besonders bevorzugt 95:5 oder mehr beträgt.

5. Verwendung einer Verbindung der Formel (I) oder (II) nach Anspruch 1 oder einer Mischung nach einem der Ansprüche 2, 3 oder 4 als Geschmacksstoff, vorzugsweise zum Erzeugen, Vermitteln, Modifizieren und/oder Verstärken eines Geschmackseindrucks, insbesondere eines Umami-Geschmacks.

6. Zusammensetzung, insbesondere zum Verzehr geeignete Zusammensetzung, bestehend aus oder umfassend
- eine geschmacklich wirksame Menge einer Verbindung der Formel (I) oder (II) nach Anspruch 1 oder einer Mischung nach einem der Ansprüche 2, 3 oder 4 und außerdem
- einen oder mehrere zum Verzehr geeignete weitere Bestandteile.

7. Zusammensetzung nach Anspruch 6, wobei die weiteren Bestandteile
a) feste Trägerstoffe oder
b) feste Trägerstoffe und eine Aromakomposition oder
c) Wasser, eine Ölphase, ein oder mehrere W/O-Emulgatoren, gegebenenfalls ein oder mehrere Antioxidantien und gegebenenfalls ein oder mehrere Stoffe zur Verstärkung einer antioxidativen Wirkung
sind.

8. Zusammensetzung nach Anspruch 6 oder 7, wobei die weiteren Bestandteile feste Trägerstoffe umfassen und das Gewichtsverhältnis der Gesamtmenge an Verbindungen der Formeln (I), (II), (III) und (IV) zur Gesamtmenge an festen Trägerstoffen bezogen auf die Trockenmasse der Zusammensetzung im Bereich von 1:10 bis 1:100000, bevorzugt im Bereich von 1:50 bis 1:20000, besonders bevorzugt im Bereich von 1:100 bis 1:5000 liegt.

9. Zusammensetzung nach Anspruch 6 oder 7, umfassend oder bestehend aus
- 0,01 bis 0,1 Gew.-% an Verbindungen der Formeln (I), (II), (III) und (IV),
- 5 bis 30 Gew.-%, bevorzugt 8 bis 25 Gew.-% Wasser,
- 50 bis 90 Gew.-%, bevorzugt 60 bis 80 Gew.-% einer Ölphase,
- 0,1 bis 5 Gew.-% eines verzehrbaren W/O-Emulgators
jeweils bezogen auf das Gesamtgewicht der Zusammensetzung
sowie
- gegebenenfalls einem oder mehreren Antioxidantien und gegebenenfalls einem oder mehreren Stoffen zur Verstärkung einer antioxidativen Wirkung.

10. Der Ernährung, der Mundpflege oder dem Genuss dienende (i) gebrauchs- oder verzehrfertige Zubereitung oder (ii) Halbfertigware, umfassend
- eine geschmacklich wirksame Menge einer Verbindung der Formel (I) oder (II) nach Anspruch 1
oder einer Mischung nach einem der Ansprüche 2, 3 oder 4,
oder
- eine Zusammensetzung nach einem der Ansprüche 6 bis 9.

11. Der Ernährung, der Mundpflege oder dem Genuss dienende (i) gebrauchs- oder verzehrfertige Zubereitung nach Anspruch 10, umfassend 0,01 ppm bis 100 ppm, bevorzugt 0,1 ppm bis 50 ppm, besonders bevorzugt 0,5 ppm bis 30 ppm an Verbindungen der Formeln (I), (II), (III) und (IV) bezogen auf das Gesamtgewicht der (i) gebrauchs- oder verzehrfertigen Zubereitung.

12. (ii) Halbfertigware nach Anspruch 10, umfassend 10 ppm bis 100000 ppm, bevorzugt 25 ppm bis 5000 ppm, besonders bevorzugt 50 ppm bis 1200 ppm an Verbindungen der Formeln (I), (II), (III) und (IV) bezogen auf das Gesamtgewicht der (ii) Halbfertigware.

13. (ii) Halbfertigware nach Anspruch 10 oder 12, umfassend
- 0 bis 10 Gew.-%, bevorzugt 0,0001 bis 5 Gew.-%, besonders bevorzugt 0,001 bis 2 Gew.-% an Natriumglutamat,
sowie
- 0 bis 90 Gew.-%, bevorzugt 0,0001 bis 90 Gew.-%, bevorzugt 0,001 bis 30 Gew.-% einer Aromakomposition
jeweils bezogen auf das Gesamtgewicht der (ii) Halbfertigware.

14. Zusammensetzung, (i) gebrauchs- oder verzehrfertige Zubereitung oder (ii) Halbfertigware nach einem der Ansprüche 6 bis 13, zusätzlich umfassend eine Substanz zum Maskieren oder Vermindern eines unangenehmen Geschmackseindrucks und/oder eine Substanz zum Verstärken eines angenehmen Geschmackseindrucks.

15. Verfahren zum Erzeugen, Vermitteln, Modifizieren und/oder Verstärken eines Geschmacks in einer der Ernährung, der Mundpflege oder dem Genuss dienenden (i) gebrauchs- oder verzehrfertigen Zubereitung oder (ii) Halbfertigware, umfassend folgenden Schritt:
- Vermischen
(A) einer geschmacklich wirksamen Menge einer Verbindung der Formel (I) oder (II) nach Anspruch 1 oder einer Mischung nach einem der Ansprüche 2, 3 oder 4 oder
(B) einer Zusammensetzung nach einem der Ansprüche 6 bis 9 oder 14 mit einem oder mehreren weiteren Bestandteilen der (i) verzehrfertigen Zubereitung oder der (ii) Halbfertigware;
oder
- Applizieren
(A) einer geschmacklich wirksamen Menge einer Verbindung der Formel (I) oder (II) nach Anspruch 1 oder einer Mischung nach einem der Ansprüche 2, 3 oder 4
oder
(B) einer Zusammensetzung nach einem der Ansprüche 6 bis 9 oder 14 auf einen oder mehrere weitere Bestandteile der (i) verzehrfertigen Zubereitung oder der (ii) Halbfertigware;
oder
- Einbetten
(A) einer geschmacklich wirksamen Menge einer Verbindung der Formel (I) oder (II) nach Anspruch 1 oder einer Mischung nach einem der Ansprüche 2, 3 oder 4 oder
(B) einer Zusammensetzung nach einem der Ansprüche 6 bis 9 oder 14 in ein Hüll- oder Matrixmaterial,
vorzugsweise zum Erzeugen, Vermitteln, Modifizieren und/oder Verstärken eines Umami-Geschmacks.

## Claims

1. Compound of formula (I) or (II)

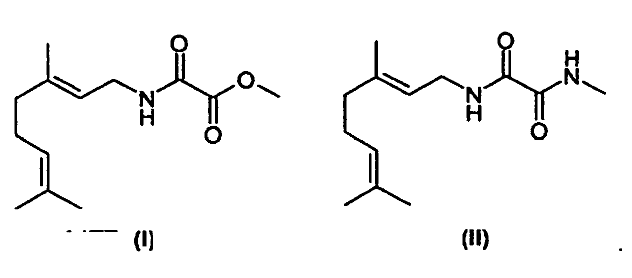

2. Mixture, in particular flavouring mixture, comprising a compound of formula (I) and/or a compound of formula (II) or consisting of a compound of formula (I) and a compound of formula (II) according to claim 1.

3. Mixture, in particular flavouring mixture, comprising or consisting of
- a compound of formula (I) according to claim 1 and a compound of formula (III) 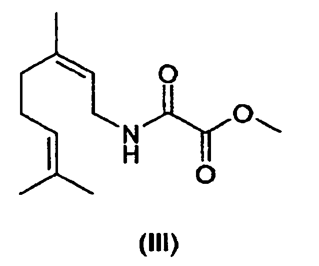 or
- a compound of formula (II) according to claim 1 and a compound of formula (IV) 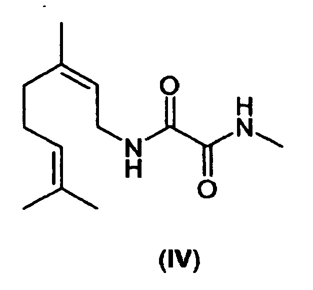 or
- all the compounds of formulae (I), (II), (III) and (IV).

4. Mixture according to claim 3, wherein the weight ratio of the total amount of compounds of formulae (I) and (II) to the total amount of compounds of formulae (III) and (IV) is 85:15 or more, preferably 90:10 or more, particularly preferably 95:5 or more.

5. Use of a compound of formula (I) or (II) according to claim 1 or of a mixture according to any one of claims 2, 3 or 4 as a flavouring, preferably for producing, imparting, modifying and/or enhancing a taste impression, in particular an umami taste.

6. Composition, in particular composition suitable for consumption, consisting of or comprising
- an amount that is effective in terms of taste of a compound of formula (I) or (II) according to claim 1 or of a mixture according to any one of claims 2, 3 or 4
and, in addition,
- one or more further constituents suitable for consumption.

7. Composition according to claim 6, wherein the further constituents are
a) solid carriers or
b) solid carriers and a flavour composition or
c) water, an oil phase, one or more W/O emulsifiers, optionally one or more antioxidants and optionally one or more substances for enhancing an antioxidative action.

8. Composition according to claim 6 or 7, wherein the further constituents include solid carriers and the weight ratio of the total amount of compounds of formulae (I), (II), (III) and (IV) to the total amount of solid carriers, based on the dry mass of the composition, is in the range from 1:10 to 1:100,000, preferably in the range from 1:50 to 1:20,000, particularly preferably in the range from 1:100 to 1:5000.

9. Composition according to claim 6 or 7, comprising or consisting of
- from 0.01 to 0.1 wt.% compounds of formulae (I), (II), (III) and (IV),
- from 5 to 30 wt.%, preferably from 8 to 25 wt.%, water,
- from 50 to 90 wt.%, preferably from 60 to 80 wt.%, of an oil phase,
- from 0.1 to 5 wt.% of a consumable W/O emulsifier,
in each case based on the total weight of the composition,
and
- optionally one or more antioxidants and optionally one or more substances for enhancing an antioxidative action.

10. (i) Ready-to-use or ready-to-eat preparation or (ii) semi-finished product for nutrition, oral care or enjoyment, comprising
- an amount that is effective in terms of taste of a compound of formula (I) or (II) according to claim 1
or of a mixture according to any one of claims 2, 3 or 4,
or
- a composition according to any one of claims 6 to 9.

11. (i) Ready-to-use or ready-to-eat preparation for nutrition, oral care or enjoyment according to claim 10, comprising from 0.01 ppm to 100 ppm, preferably from 0.1 ppm to 50 ppm, particularly preferably from 0.5 ppm to 30 ppm, of compounds of formulae (I), (II), (III) and (IV), based on the total weight of (i) the ready-to-use or ready-to-eat preparation.

12. (ii) Semi-finished product according to claim 10, comprising from 10 ppm to 100,000 ppm, preferably from 25 ppm to 5000 ppm, particularly preferably from 50 ppm to 1200 ppm, of compounds of formulae (I), (II), (III) and (IV), based on the total weight of (ii) the semi-finished product.

13. (ii) Semi-finished product according to claim 10 or 12, comprising
- from 0 to 10 wt.%, preferably from 0.0001 to 5 wt.%, particularly preferably from 0.001 to 2 wt.%, sodium glutamate,
and
- from 0 to 90 wt.%, preferably from 0.0001 to 90 wt.%, preferably from 0.001 to 30 wt.%, of a flavour composition,
in each case based on the total weight of (ii) the semi-finished product.

14. Composition, (i) ready-to-use or ready-to-eat preparation or (ii) semi-finished product according to any one of claims 6 to 13, additionally comprising a substance for masking or reducing an unpleasant taste impression and/or a substance for enhancing a pleasant taste impression.

15. Method for producing, imparting, modifying and/or enhancing a taste in (i) a ready-to-use or ready-to-eat preparation or (ii) a semi-finished product for nutrition, oral care or enjoyment, comprising the following step:
- mixing
(A) an amount that is effective in terms of taste of a compound of formula (I) or (II) according to claim 1 or of a mixture according to any one of claims 2, 3 or 4 or
(B) a composition according to any one of claims 6 to 9 or 14 with one or more further constituents of (i) the ready-to-eat preparation or (ii) the semi-finished product;
or
- applying
(A) an amount that is effective in terms of taste of a compound of formula (I) or (II) according to claim 1 or of a mixture according to any one of claims 2, 3 or 4 or
(B) a composition according to any one of claims 6 to 9 or 14 to one or more further constituents of (i) the ready-to-eat preparation or (ii) the semi-finished product;
or
- embedding
(A) an amount that is effective in terms of taste of a compound of formula (I) or (II) according to claim 1 or of a mixture according to any one of claims 2, 3 or 4 or
(B) a composition according to any one of claims 6 to 9 or 14 in a casing or matrix material,
preferably for producing, imparting, modifying and/or enhancing an umami taste.

## Revendications

1. Composé de la formule (I) ou (II)

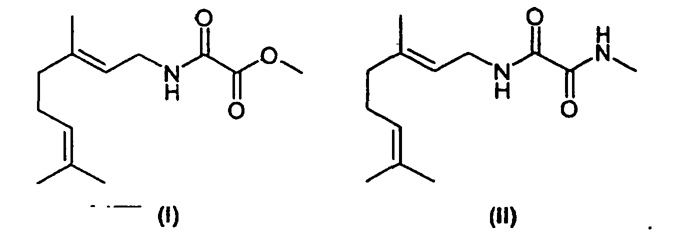

2. Mélange, en particulier mélange d'aromates, comprenant un composé de la formule (I) et/ou un composé de la formule (II), ou consistant en un composé de la formule (I) et un composé de la formule (II) selon la revendication 1.

3. Mélange, en particulier mélange d'aromates, comprenant ou consistant
- en un composé de la formule (I) selon la revendication 1, ainsi qu'en un composé de la formule (III) 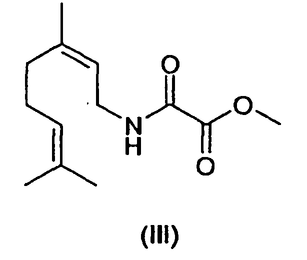 ou
- en un composé de la formule (II) selon la revendication 1, ainsi qu'en un composé de la formule (IV) 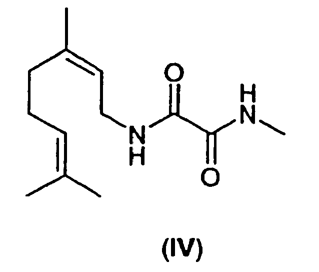 ou
- en tous les composés des formules (I), (II), (III) et (IV).

4. Mélange selon la revendication 3, dans lequel les rapports pondéraux de la quantité totale des composés des formules (I) et (II) à la quantité totale des composés des formules (III) et (IV) est de 85 : 15 ou plus, de préférence de 90 : 10 ou plus, de façon particulièrement préférée de 95 : 5 ou plus.

5. Utilisation d'un composé de la formule (I) ou (II) selon la revendication 1 ou d'un mélange selon l'une quelconque des revendications 2, 3 ou 4 comme aromate, en particulier pour produire, communiquer, modifier et/ou renforcer une impression de goût, en particulier d'un goût umami.

6. Composition, en particulier composition appropriée à la consommation, consistant en ou comprenant
- une quantité gustativement efficace d'un composé des formules (I) ou (II) selon la revendication 1 ou d'un mélange selon l'une quelconque des revendications 2, 3 ou 4
et en outre
- un ou plusieurs autres composants appropriés à la consommation.

7. Composition selon la revendication 6, les autres composants étant
a) des matériaux supports solides ou
b) des matériaux supports solides et une composition d'aromates ou
c) de l'eau, une phase huileuse, un ou plusieurs émulsionnants eau/huile, le cas échéant un ou plusieurs antioxydants et, le cas échéant, une ou plusieurs substances pour renforcer un effet antioxydant.

8. Composition selon la revendication 6 ou 7, les autres composants comprenant des substances supports solides et le rapport pondéral de la quantité totale de composés des formules (I), (II), (III) et (IV) à la quantité totale des substances supports solides, rapporté à la masse sèche de la composition se trouvant dans l'intervalle de 1 : 10 à 1 : 100000, de préférence dans l'intervalle de 1 : 50 à 1 20000, de façon particulièrement préférée dans l'intervalle de 1 : 100 à 1 : 5000.

9. Composition selon la revendication 6 ou 7, comprenant ou consistant en
- 0,01 à 0,1 % en poids de composés des formules (I), (II), (III) et (IV),
- 5 à 30 % en poids, de préférence 8 à 25 % en poids d'eau,
- 50 à 90 % en poids, de préférence 60 à 80 % en poids d'une phase huileuse,
- 0,1 à 5 % en poids d'un émulsionnant eau/huile comestible,
rapportés dans chaque cas au poids total de la composition,
ainsi que
- le cas échéant un ou plusieurs antioxydants et le cas échéant une ou plusieurs substances pour renforcer l'effet antioxydant.

10. Composition (i) prête à l'emploi ou à la consommation ou produit semi-fini (ii) servant à l'alimentation, aux soins de la bouche ou à la stimulation, comprenant
- une quantité gustativement efficace d'un composé de la formule (I) ou (II) selon la revendication 1
ou un mélange selon l'une quelconque des revendications 2, 3 ou 4
ou
- une composition selon l'une quelconque des revendications 6 à 9.

11. Composition (i) prête à l'emploi ou à la consommation servant à l'alimentation, aux soins de la bouche ou à la stimulation selon la revendication 10, comprenant 0,01 ppm à 100 ppm, de préférence 0,1 ppm à 50 ppm, de façon particulièrement préférée 0,5 ppm à 30 ppm de composés des formules (I), (II), (III) et (IV), rapportés au poids total de la composition (i) prête à l'emploi ou à la consommation.

12. Produit semi-fini (ii) selon la revendication 10, comprenant 10 ppm à 100000 ppm, de préférence 25 ppm à 5000 ppm, de façon particulièrement préférée 50 ppm à 1200 ppm de composés des formules (I), (II), (III) et (IV), rapportés au poids total du produit semi-fini (ii).

13. Produit semi-fini (ii) selon la revendication 10 ou 12, comprenant
- 0 à 10 % en poids, de préférence 0,0001 à 5 % en poids, de façon particulièrement préférée 0,001 à 2 % en poids de glutamate de sodium,
ainsi que
- 0 à 90 % en poids, de préférence 0,0001 à 90 % en poids, de préférence 0,001 à 30 % en poids d'une composition d'aromates
rapportés à chaque fois au poids total du produit semi-fini (ii).

14. Composition, composition (i) prête à l'emploi ou à la consommation ou produit semi-fini (ii) selon l'une quelconque des revendications 6 à 13, comprenant en plus une substance pour masquer ou atténuer une impression gustative désagréable et/ou une substance pour renforcer une impression gustative agréable.

15. Procédé pour produire, communiquer, modifier et/ou renforcer une saveur dans une composition (i) prête à l'emploi ou à la consommation ou dans un produit semi-fini (ii) servant à l'alimentation, aux soins de la bouche ou à la stimulation, comprenant les étapes suivantes :
- mélangeage
(A) d'une quantité gustativement efficace d'un composé de la formule (I) ou (II) selon la revendication 1 ou d'un mélange selon l'une quelconque des revendications 2, 3 ou 4
ou
(B) d'une composition selon l'une quelconque des revendications 6 à 9 ou 14 avec un ou plusieurs autres composants de la composition (i) prête à la consommation ou du produit semi-fini (ii);
ou
- application
(A) d'une quantité gustativement efficace d'un composé de la formule (I) ou (II) selon la revendication 1 ou d'un mélange selon l'une quelconque des revendications 2, 3 ou 4
ou
(B) d'une composition selon l'une quelconque des revendications 6 à 9 ou 14 sur un ou plusieurs autres composants de la composition (i) prête à la consommation ou du produit semi-fini (ii) ;
ou
- incorporation
(A) d'une quantité gustativement efficace d'un composé de la formule (I) ou (II) selon la revendication 1 ou d'un mélange selon l'une quelconque des revendications 2, 3 ou 4
ou
(B) d'une composition selon l'une quelconque des revendications 6 à 9 ou 14 dans un matériau d'enrobage ou une matrice,
de préférence pour produire, communiquer, modifier et/ou renforcer une saveur umami.
